(19) Europäisches Patentamt
European Patent Office
Office européen des brevets

(11) EP 4 382 529 A1

(12) EUROPEAN PATENT APPLICATION

(43) Date of publication:
**12.06.2024 Bulletin 2024/24**

(21) Application number: **22211952.1**

(22) Date of filing: **07.12.2022**

(51) International Patent Classification (IPC):
***C07D 487/04*** (2006.01) ***C07D 207/12*** (2006.01)

(52) Cooperative Patent Classification (CPC):
**C07D 487/04; C07D 207/12**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Bayer Consumer Care AG
4052 Basel (CH)**

(72) Inventor: **The designation of the inventor has not yet been filed**

(74) Representative: **BIP Patents
c/o Bayer Intellectual Property GmbH
Alfred-Nobel-Straße 50
40789 Monheim am Rhein (DE)**

# (54) A PROCESS FOR PREPARING PURE (3S)-PYRROLIDIN-3-OL AND PURE (3S)-PYRROLIDIN-3-OL HYDROCHLORIDE

(57) The present invention relates to a process for preparing of pure (3S)-Pyrrolidin-3-ol ("the base") of formula (I)

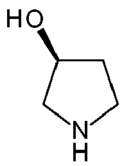

(I)

and the preparation of the corresponding (3S)-Pyrrolidin-3-ol hydrochloride ("the hydrochloride") of formula (II)

EP 4 382 529 A1

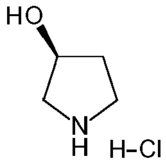
(II)

## Description

### Summary of invention

The present invention relates to a process for preparing of pure (3S)-Pyrrolidin-3-ol ("the base") of formula (I)

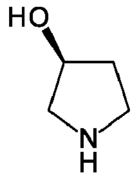

(I)

and the preparation of the corresponding (3S)-Pyrrolidin-3-ol hydrochloride ("the hydrochloride") of formula (II)

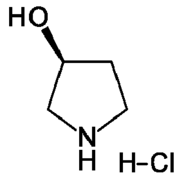

(II)

Larotrectinib (formula (IIIa)) is a novel active ingredient used in oncology,

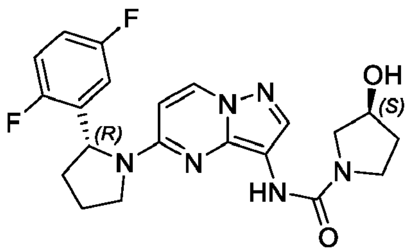

(IIIa)

which is contained in the pharmaceutical drug Vitrakvi® approved in Europe, the United States and many other countries.

The present invention is also directed to pure (3S)-pyrrolidin-3-ol ("the base") of formula (I) and pure (3S)-Pyrrolidin-3-ol hydrochloride ("the hydrochloride") of formula (II).

The present invention is also directed to the use of pure (3S)-Pyrrolidin-3-ol ("the base") of formula (I) or pure (3S)-Pyrrolidin-3-ol hydrochloride ("the hydrochloride") of formula (II) according to the invention for the preparation of Larotrectinib or a salt thereof.

The present invention is also directed to a process for preparing pure Larotrectinib or a salt thereof, by using pure (3S)-Pyrrolidin-3-ol ("the base") of formula (I) or pure (3S)-Pyrrolidin-3-ol hydrochloride ("the hydrochloride") of formula (II) according to the invention.

The present invention is also directed to a process for preparing pure Larotrectinib or a salt thereof, by using pure (3S)-Pyrrolidin-3-ol ("the base") of formula (I) or pure (3S)-Pyrrolidin-3-ol hydrochloride ("the hydrochloride") of formula (II) prepared according to the invention.

### Definitions

**Salt:** Salt is a chemical compound consisting of an ionic assembly of positively charged cations and negatively charged anions. Non-limiting examples for cations are Calcium ($Ca^{2+}$,), Magnesium ($Mg^{2+}$), Sodium ($Na^+$), Potassium ($K^+$), Lithium ($Li^+$) and Aluminum ($Al^{3+}$). Non-limiting examples for anions are Chloride ($Cl^-$), Oxide ($O^{2-}$), Hydroxide ($OH^-$), Phosphates ($PO_4^{3-}$, $HPO_4^{2-}$, $H_2PO_4^-$) and Sulfates ($SO_4^{2-}$, $HSO_4^-$).

**Substantially free:** compound Y shall be defined "to be substantially free of impurity X" if impurity X cannot be detected in a sample of compound Y or only in an amount less than 0.01 % (area-%) with the method disclosed for

the content determination of compound Y and impurity X.

The drug product Vitrakvi® is on the markets in form of capsules which contain a sulfate salt of Larotrectinib according to formula (IIIb) or in form of a liquid formulation manufactured from this sulfate salt.

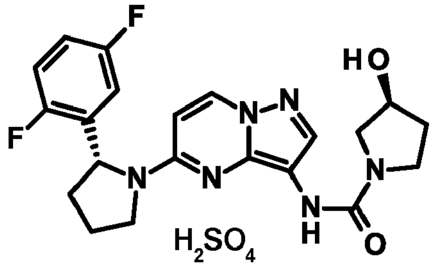
(IIIb)

Larotrectinib (IIIa) and the sulfate salt (IIIb) were first disclosed in examples 14 and 14 A of WO2010/048314A1.

An important structural element of Larotrectinib is (3S)-Pyrrolidin-3-ol (I) which is connected to the rest of the molecule via isocyanate condensation. (3S)-Pyrrolidin-3-ol (I) can be used as the free base of formula (I) or as the hydrochloride of formula (II), which is converted into the free base in situ (e.g. by a stronger base such as sodium hydroxide, or an organic base). The free base (I) and the hydrochloride (II) are therefore extremely important for an inexpensive process for preparing Larotrectinib.

An improved synthesis of Larotrectinib or a salt thereof as used for the market product Vitrakvi® is disclosed in WO2017201241A1.

Larotrectinib of formula (IIIa)

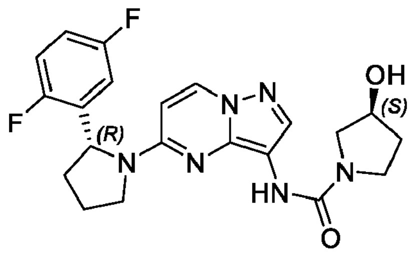
(IIIa)

or a salt thereof,
is obtained by treating a compound of formula 13

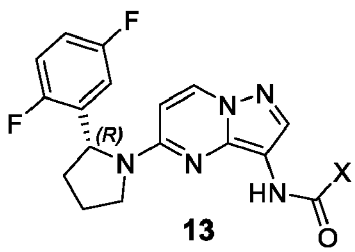
13

or a salt thereof, wherein X is phenoxy
with (3S)-Pyrrolidin-3-ol ("the base") of formula (I)

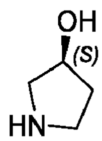
(I)

or a salt thereof to form Larotrectinib or a salt thereof.

Since this reaction step is in the 'Final Step Window' of GMP production, particularly high requirements have been placed on the quality of (3S)-pyrrolidin-3-ol ("the base") of formula (I) and (3S)-Pyrrolidin-3-ol hydrochloride ("the hydrochloride") as key intermediate.

It is therefore especially important that the free base of formula (I) or the hydrochloride of formula (II) are of high optical and chemical purity.

Furthermore, a process must be available that is scalable on the one hand and inexpensive on the other. It must be ensured that safety-relevant aspects are met and that the high quality is maintained when the process is scaled up to commercial production.

This is guaranteed by the present process for preparing pure (3S)-pyrrolidin-3-ol ("the base") of formula (I) and pure (3S)-Pyrrolidin-3-ol hydrochloride ("the hydrochloride") of formula (II).

(3S)-Pyrrolidin-3-ol (and also its hydrochloride (II)) are not novel and have already been described in numerous publications.

There are also numerous processes to synthesize the free base of formula (I), all of which, however, are unsuitable for up-scaling, or are only inadequately disclosed to the person skilled in the art, so that reworking and assessment are difficult.

Examples are: intermediate for Calcium antagonist (Barnidipine) (European Patent Laid-Open Publication No. 160, 451; J. Med. Chem. 1986, 29, 2504-2511; Japanese Patent Laid-Open Publication No. (Sho) 61-267577; Japanese Patent Laid-Open Publication No. (Sho) 61-63652); carbapenem antibiotics (Heterocycles, vol 24, No 5, 1986; Tetrahedron Lett. , 25, 2793, 1984; International Publication No. WO88/08845; J. Org. Chem. 1992, 57, 4352-4361); quinolone-based antibiotics (U. S. Pat. No. 4, 916, 141; European Patent Laid-Open Publication No. 391, 169; European Patent laid-Open Publication No. 304, 087), analgesics (K-receptor agonists) (European Patent Laid-Open Publication Nos. 398, 720 and 366, 327; J. Med. Chem. , 1994, 37, 2138-2144), and a major intermediate raw material of a neurotransmitter (International Publication No. WOOI/ 19817). (JP2001220372; WO97/43256; JP05255204; Synlett, 1995, 55-57; Syn. Comm. 1994, 24, 1381-1387; Korean J. of Med. Chem. 1993, 3, 72-80; Syn. Comm. 1993, 23, 2691-2699; J. Chem. Soc. Perkin Trans. 1. 1993, 1421-1424; Bioorganic & Medicinal Chemistry Letters, 2, 827). (Syn. Commun. 15, 587-598, 985; J. Med. Chem. 1994, 37, 2138-2144). (WO2003/097594), (WO2000/015610). (JP60104061); (JP57056457); (EP431, 521 and EP347, 818).

A close process is described in US 7, 652, 152 B2 of the company Chiroad Incorporate, Daejeon (KR), since it pursues a very similar synthesis strategy, but for an up-scaling from a safety point of view, as well as the toxicological point of view, as it corresponds to the requirements for a GMP intermediate of the pharmaceutical industry, is unsuitable. No final yields or analytical methods for verifying the purity (enantiomeric excess and chemical purity) are given. These are important parameters to judge whether this process is suitable for up-scaling a GMP synthesis and fulfills analytical requirements.

The synthesis begins with the production of the methyl ester (Va) starting from the aminohydroxybutyric acid (IV) by esterification in methanol with sulfuric acid (step (va)).

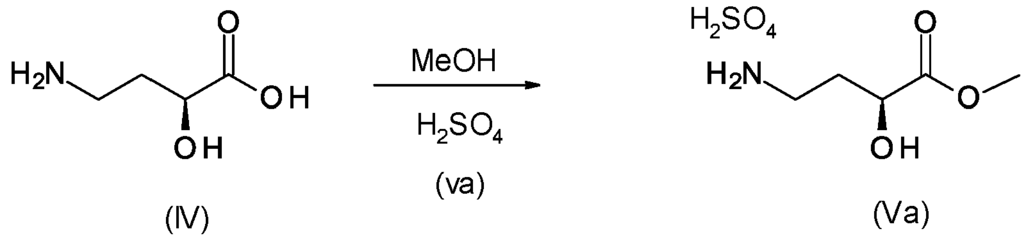


(IV) (Va)

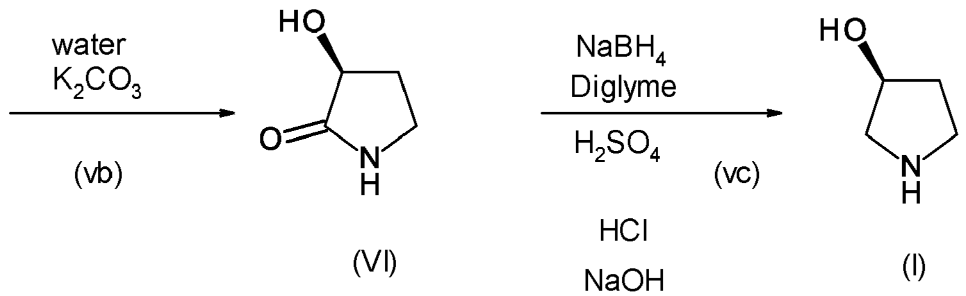


(VI) (I)

It is known that when the reaction is carried out in this way, carcinogenic sulfuric acid esters with methanol can be formed, which can be carried over into subsequent steps of the synthesis. This is to be viewed critically as the ester is not isolated and is immediately further converted to the lactam (VI).

Then, water and potassium carbonate are added, in step (vb). This can be entirely feasible in the laboratory, but in a production facility there would be strong foaming due to the formation of carbon dioxide, which would mean that the potassium carbonate would have to be added in many small portions in order to control gas formation and for the complete neutralization of the sulfuric acid, this is very disadvantageous from a production point of view.

For work-up, methanol is added, and the salts are filtered off twice. Aqueous methanol can still dissolve some potassium sulphate, unfortunately nothing of this is mentioned, instead everything is then concentrated to dryness.

Such a procedure is unsuitable for up-scaling in a production plant, since the product is isolated by default using an isolation device such as a pressure filter / suction filter or a centrifuge, but this is not possible because no solvent for isolation via a crystalline intermediate is disclosed.

In the subsequent step (vc) for the preparation of (3S)-Pyrrolidin-3-ol (I), the lactam is treated with 4 eq. sodium borohydride in diglyme, then added concentrated sulfuric acid at 25 °C and heated to 80 °C for 12 hours.

In this reaction, the highly explosive diborane is released in situ, since a large excess of sodium borohydride is used, a great deal of diborane is formed in the reaction vessel (also in the gas phase). Unfortunately, it has happened that the uncontrolled handling of diborane can and has already led to strong explosions (Bretherick's Handbook of Reactive Chemical Hazards). Therefore, upscaling such a reaction is not reasonable in terms of security. Furthermore, with such a reaction, the formation of highly carcinogenic aziridines can be assumed. Aziridines are known to form from the sulfuric acid half-ester after treatment with base, in this case 10N sodium hydroxide solution pH 11. The formation of an analogous aziridine is described in Synthesis, (20), 3423-3428; 2010 and Journal of Organic Chemistry, 32 (8), 2388-91; 1967.

How such secondary components are separated has unfortunately not been disclosed.

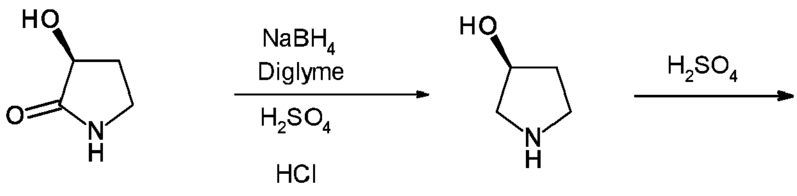

(VI) (I)

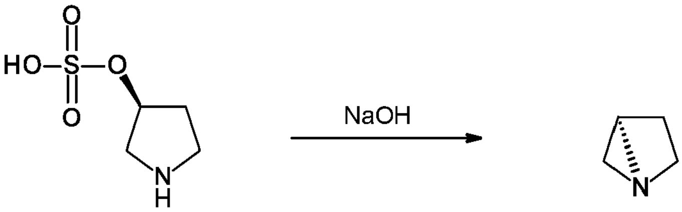

(Ia) (Ib)

Salts are filtered off again and the filtrate is concentrated.

It is known that salts are also dragged into the filtrate at this point, which would greatly impair a subsequent technical distillation (e.g. falling-film evaporator / thin-film evaporator). No yield is given in the example described.

Viewed as a whole, the disclosures given in US Pat. No. 7,652,152 B2, are very poor and not suitable for upscaling to the industrial scale; there is no statement about an overall yield as well as the final quality achieved.

The object of the present invention is to provide a process to obtain pure (3S)-Pyrrolidin-3-ol of formula (I) and pure (3S)-Pyrrolidin-3-ol hydrochloride of formula (II) which is scalable, cheap and safe, can be carried out under GMP, in which crystalline intermediates are run through, which are easy to isolate and which enable to obtain the free base and its hydrochloride with a very high optical and chemical purity which is a prerequisite for industrial pharmaceutical production and required by regulatory authorities.

The pure (3S)-Pyrrolidin-3-ol of formula (I) shall have

- a chemical purity determined by GC according to method (I) A of not less than 99.80% (area-%)
- an optical purity determined by GC according to method (I) B of not less than 99.9% (enantiomeric excess %ee)
- a content on anhydrous basis determined by potentiometric titration and Karl-Fischer titration according to method

(I) C of not less than 99.0 % (m/m%)

and shall contain

- unspecified impurities determined by GC according to method (I) A in an amount less than 0.10% (area-%))
- (S)-Pyrrolidin-3-ol open ring dimer of formula (IX)

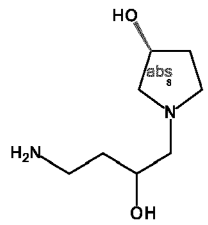

(XI)

determined by GC according to method (I) A in an amount less than 0.05 % (area-%)

- 4-Amino-1,2-butanediol of formula (XII)

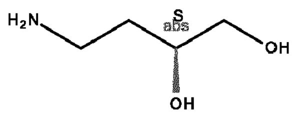

(XII)

determined by GC according to method (I) A in an amount less than 0.05 % (area-%)

The pure (3S)-Pyrrolidin-3-ol hydrochloride of formula (II) shall have

- a chemical purity determined by GC according to method (II) A of not less than 99.5% (area-%)
- an optical purity determined by GC according to method (II) B of not less than 99.9% (enantiomeric excess, %ee)

The process of the present invention is surprisingly able to meet all the above requirements and solve the problem.

The process of the present invention for preparing pure (3S)-Pyrrolidin-3-ol of formula (I) and pure (3S)-Pyrrolidin-3-ol hydrochloride of formula (II) has proven to be a particularly efficient, cost-effective and scalable process that can be carried out under GMP, in which crystalline intermediates are run through that are easy to isolate and that enable to meet the high purity requirements in terms of optical and chemical purity, which is a prerequisite for industrial pharmaceutical production and required by regulatory authorities. The overall yield (based on the average yield per step) starting from aminohydroxybutyric acid (IV) is high, 44 % Th. over the 4 steps (a) to (d).

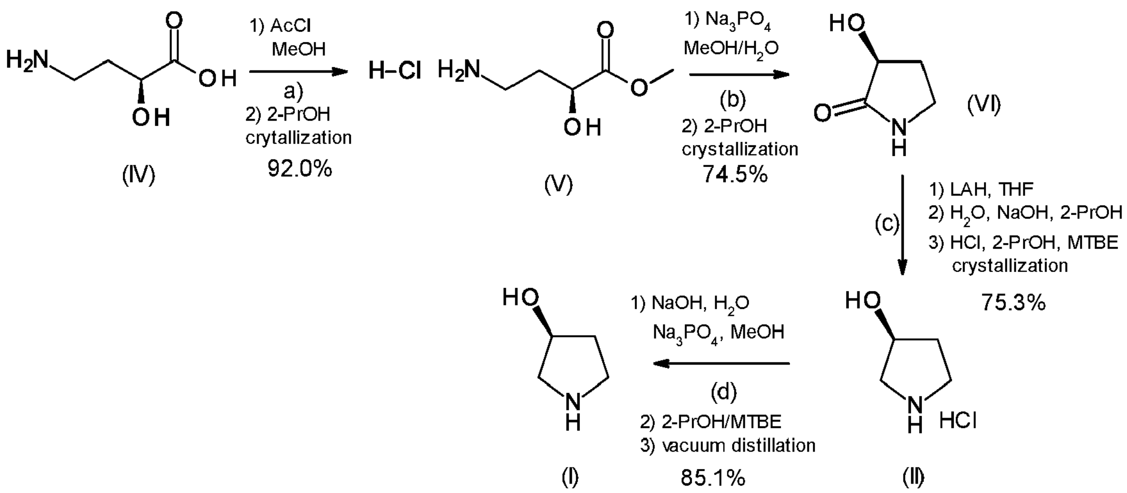

No chemical contamination and no genotoxic by-products are found. This also distinguishes the process of the present invention from the processes described in the prior art. The process of the present invention meets all the requirements for the specification that the conditions for the approval of a medicinal product in the various countries must meet. The new inventive process is currently used successfully in the commercial product manufacture of Larotrectinib.

Step a

The Methylester hydrochloride (V)

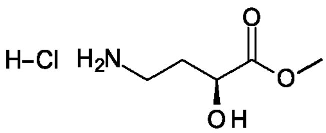

(V)

is made in step (a) from the aminohydroxybutyric acid (IV)

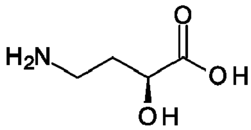

(IV)

by acid-catalyzed esterification with methanol and in situ salination with HCl to form the hydrochloride. For this purpose, the aminohydroxybutyric acid (IV) is suspended in 6-15 fold, methanol, preferably 8-12 fold, most preferably 10 fold and then heated to 55-70 °C, preferably 55-65 °C, most preferably at 55-60 °C and then treated with 1.8 - 3 eq. acetyl chloride preferably 1.8-2.2 eq., most preferably 2.0-2.1 eq. Then the mixture is refluxed for 2-4 h, preferably 2.5-3.5 h. Most of the solvent is distilled off and redistilled to isopropanol by changing the solvent. After the isopropanol suspension has been concentrated, it is cooled, and the product is isolated and dried (dried under reduced pressure at 50°C maximum jacket temperature at 12-24 h, up to 48 hours.). The yields are 81.7 to 97.0 % of theory, the chemical purity is usually ≥ 97.3% (m/m). The preparation of the methylester (V) is described in the literature, unfortunately, there is no detailed description of a safe process for scale-up and the impact on impurity profile: Gai, Yongkang; et al, Bioconjugate Chemistry (2016), 27(3), 515-520; Zeng, Dexing; et al, WO2017192605 A1 2017-11-09; Zeng, Dexing; et al, WO2017192598 A1 2017-11-09; Wang, Guangyi; et al, WO2015026792 A1 2015-02-26; Aebi, Johannes; et al, US20110092698 A1 2011-04-21; Cheshire, David; et al, WO2001062714 A1 2001-08-30; Zhang, Wen, China, CN1869008 A 2006-11-29; Noh, Gyeong Rok; et al, Korea, Republic of, KR915666 B1 2009-09-04; Aebi, Johannes; et al, US20110092698 A1 2011-04-21; Roh, Kyoung Rok; et al, WO2007011162 A1 2007-01-25. The aminohydroxybutyric acid (IV) is commercially available. Its preparation is described in the literature: Srairi, Driss; et al Bulletin de la Societe Chimique de France (1987), (2), 297-301; Wong, Chi Huey; et al. Journal of Organic Chemistry (1985), 50(11), 1992-4; Takahashi, Satomi; et al. European Patent Organization, EP430234 A2 1991-06-05; Xiao, Xun; et al. China, CN104098502 A 2014-10-15.

Step (b)

The Lactam (VI)

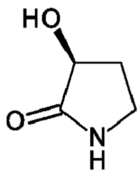

(VI)

is obtained in step (b) by a base-induced ring closure reaction, starting from the methyl ester hydrochloride (V)

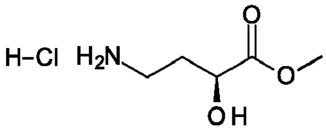

(V)

For this purpose, the methyl ester hydrochloride is dissolved in a protic solvent such as methanol, ethanol, isopropanol, but preferably in 8-12-fold methanol, preferably 10.5-11.5 fold are used. Sodium phosphate has proven particularly useful as a base, and here in particular the anhydrous form of the salt, here 0.8- 1.5 eq., preferably 1.0 - 1.38 eq., and most preferably 1.1-1.2 eq used. The reaction time is 4-8 h, preferably 4-6 h, one works at 20-60 °C, preferably at 40-55 °C. To work up the batch, water is added and then the dodecahydrate of sodium phosphate is filtered off from the mixture. The filtrate is redistilled to isopropanol and azeotroped by adding isopropanol until a water value (KF: Karl Fischer) of < 2% (m/m) is reached. The salts are then filtered off a second time and, after further concentration at low temperature, -13°C to 0°C, preferably at -10°C. The product is crystallized, isolated and dried (under reduced pressure at max. 50°C jacket temperature for 12-24 h, up to max. 72 h). The yields are in the range of 61.6 to 82.4 % of theory, the chemical purity is usually ≥ 98.3% (m/m).

The synthesis of lactam (VI) is described in the literature: Rong, Hao-Jie; et al, Journal of Organic Chemistry (2017), 82(1), 532-540; Davies, Christopher D.; et al, Synlett (2008), (13), 2028-2032; Jiang, Chaozhe; et al, Cuihua Xuebao (2005), 26(4), 263-264; Gao, Renlong; et al, WO2015043364 A1 2015-04-02; Olejniczak, Jason; et al, Macromolecules (Washington, DC, United States) (2015), 48(10), 3166-3172; Almutairi, Adah; et al, WO2016164828 A1 2016-10-13; Bjornson, Kyla; et al, WO2014008285 A1 2014-01-09; Kharul, Rajendra; et al, WO2011013141 A2 2011-02-03; Srairi, Driss; et al, Bulletin de la Societe Chimique de France (1987), (2), 297-301; Roh, Kyoung Rok; et al, WO2007011162 A1 2007-01-25; Huang, Pei-Qiang; et al, Heterocycles (2003), 60(8), 1833-1841; Bentley, Jonathan M.; et al, Journal of the Chemical Society, Chemical Communications (1995), (2), 231-2; Bersot, Ross; et al, United States, US20150284362 A1 2015-10-08; Bjornson, Kyla; et al, WO2014008285 A1 2014-01-09; Kharul, Rajendra; et al, WO2011013141 A2 2011-02-03; Pires, Raul; et al, Tetrahedron (1997), 53(27), 9213-9218; Olejniczak, Jason et al, Macromolecules (Washington, DC, United States) (2015), 48(10), 3166-3172; Kamal, Ahmed; et al, Tetrahedron: Asymmetry (2003), 14(17), 2587-2594. All these processes which are described in the literature are not feasible regarding scale-up of the process according to pharmaceutical requirements and with respect for the high quality of the lactam which is needed in this GMP step.

Step c)

Pure (3S)-Pyrrolidin-3-ol hydrochloride ("the hydrochloride") of formula (II)

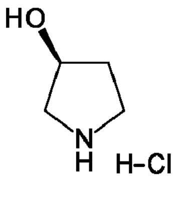

(II)

is prepared in step (c) from the lactam (VI)

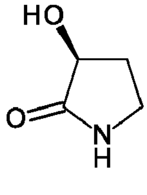

(VI)

by reducing the amide function with lithium aluminum hydride.

The reduction is preferably carried out in tetrahydrofuran (THF) as the solvent. Preference is given to 8-15 times THF regarding lactam (VI), particularly preferably 10-12 times solvents are used. The reaction time is 48-96 hours, but preferably 48-72 h, but particularly preferably 55-60 h. The lithium aluminum hydride is preferably used as a commercial solution of 2.5 M in THF. The addition is dose-controlled and can be controlled via gas evolution and internal

temperature. 3.0 to 3.5 eq. are used, preferably 3.1-3.3 eq., particularly preferably 3.2 eq.

The dosage can be performed at -10 to +40°C, preferably at 25-30°C. When the reaction is complete, water, 15% aqueous sodium hydroxide solution and again water are added, and the aluminum salts are removed by filtration and washed. The (3S)-Pyrrolidin-3-ol (I) is present up to this process step. (3S)-Pyrrolidin-3-ol hydrochloride (II) is then obtained with 5-6 N HCl in isopropanol. This is done by adding 1.0 - 1.6 eq. HCl, preferably 1.03-1.54 eq., particularly preferably 1.15-1.4 eq. in form of 5-6 N HCl in isopropanol. For quantitative salt formation, MTBE is added. The solution is cooled, and the crude product is isolated. The crude product obtained in this way has proven to be advantageous, which usually has an optical purity of approximately 99.8-99.9% (enantiomeric excess %ee) and must be dissolved again in isopropanol, 5-6 N HCl in isopropanol is added, 0.05-0.2 eq., preferably 0.09 - 0.15 eq. HCl and then add MTBE. After cooling, isolation and drying (under reduced pressure at max. T=50°C for 6-12 h, at least for 72h) yields of 71.8 to 79.6 % of theory are obtained. The product has a chemical purity of > 99.5% (area-%) and an optical purity of > 99.9%ee (enantiomeric excess)

Step (d)

The process of the present invention is primarily characterized by the desalination step (d) starting from pure (3S)-Pyrrolidin-3-ol hydrochloride ("the hydrochloride") of formula (II)

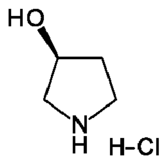
(II)

to obtain pure (3S)-Pyrrolidin-3-ol ("the base") of formula (I)

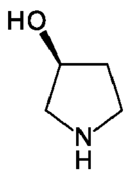
(I)

In this desalination step, inorganic bases such as NaOH, KOH, $Ca(OH)_2$, potassium carbonate, sodium carbonate, sodium phosphate, potassium phosphate or mixtures thereof can be used. However, preference is given to using NaOH, KOH and sodium phosphate or potassium phosphate and mixtures thereof. NaOH and sodium phosphate and a mixture thereof are particularly preferred.

It has been found, surprisingly, that a stoichiometric amount of inorganic base is not sufficient to obtain the salt-free (3S)-Pyrrolidin-3-ol ("the base") of formula (I).

If the ratio ((3S)-Pyrrolidin-3-ol hydrochloride) to (inorganic base) is exactly 1:1, larger proportions of the hydrochloride are found in the distillate after distillation of the crude product, which was carried over by sublimation.

Therefore, in the case of NaOH and KOH, ratios of more than 1.0 eq. need to be used, preferably 1.1-1.4 eq. particularly preferably 1.1-1.2 eq. In the case of sodium phosphate and potassium phosphate, the ratio needs to be 1.5-2.5 eq., preferably 1.6-2 eq., particularly preferably 1.6-1.7 eq.

It has been proven particularly advantageous to use mixtures of NaOH with sodium phosphate or KOH with potassium phosphate. Mixtures of 0.8-0.95 eq. NaOH or KOH and 0.7-1.8 eq. sodium phosphate or potassium phosphate, preferably 0.9-0.95 eq. NaOH or KOH and 0.8-1 eq. sodium phosphate or potassium phosphate, particularly preferably 0.9-0.95 eq. NaOH or KOH and 0.9-1.0 eq. sodium phosphate or potassium phosphate is used. 0.9 eq. NaOH and 1.0 eq sodium phosphate are very particularly preferred. The inorganic bases can be used in solid form, as hydrates or else as aqueous solutions.

The desalination is carried out in protic or aprotic organic solvents or their mixtures with water. The following solvents can be used for this purpose: methanol, ethanol, 1-propanol, 2-propanol, 1-butanol, tetrahydrofuran, 2-methyltetrahydrofuran, dioxane, dimethoxyethane, glycol, preference is given to methanol, ethanol, 1-propanol, 2-propanol, especially preferably methanol, ethanol, 2-propanol, very particularly preferably methanol. In principle, the desalination can also be carried out in pure water.

The amount of solvent based on the hydrochloride used is 5-15 times (x times means x times the volume of

solvent for 1kg substance, e.g. 10 times means: 1 kg in 10L solvent), preferably 5-10 times, particularly preferably 8-10 times.

The desalination takes place at temperatures of 0-100 °C, preferably 20-60 °C, particularly preferably 40-60°C. Very particularly preferably at 50-60°C.

After the desalination has ended, the (3S)-Pyrrolidin-3-ol (I) is isolated as a crude product for subsequent distillation. For this it is necessary to separate off the excess salts from (3S)-Pyrrolidin-3-ol (I). This is done in such a way that, for example, any water present is first azeotropically distilled off, preferably ethanol or isopropanol is used for this purpose, particularly preferably ethanol or isopropanol, is distilled again by adding solvent in order to keep the water content as low as possible, then the first part of the salts filtered off and then subsequently redistilled to another solvent, preferably isopropanol, and water is azeotropically distilled off to such an extent that a water value of <0.5% (m/m) (Karl Fischer) is reached. Typically, the final volume is about 4-7 L per kg of the (3S)-Pyrrolidin-3-ol (I) in isopropanol. Then an anti-solvent such as methyl tert-butyl ether, 2-methyl-THF, methylcyclopentyl ether, diisopropyl ether is added, but preferably methyl tert-butyl ether (MTBE) is used. It is preferred to add 3-6 times, particularly preferably 4-5 times, very preferably 4 times. The mixture is cooled to -10 to 20 °C, preferably -5 to 10 °C, particularly preferably 0-5 °C, and the salts are filtered off a second time. The filtrate is distilled at normal pressure or reduced pressure until an almost solvent-free oil remains. The isopropanol content in the crude product is usually <1%. A sulphate ash determination at this point shows that the values are always <0.05% (m/m) of ash residue. The almost quantitative removal of inorganic salts is particularly important when using short-path distillation systems (thin-film evaporators), as otherwise salts will be deposited on the walls and impair the function of the wipers. In principle, desalination could also be carried out on ion exchangers. A vacuum distillation is carried out for further purification of the product. Distillation can be carried out in the batch process, conditions approx. 2-3 mbar / 115-120 °C. However, a short-path distillation system is preferably used for distillation that is gentle on the product. Additional product can be obtained by re-distillation of the distillation sump using the same parameters. A yield of 80.1 to 87.9 % th. was obtained, this corresponds to an average yield of 84.4 % th.. The chemical purity is > 99.80% (area-%), the optical purity is > 99.9%ee (enantiomeric excess). A material produced in this way meets all the requirements of a commercial product specification and can be used directly to produce Larotrectinib.

One embodiment of the present invention is the process for preparing pure (3S)-Pyrrolidin-3-ol of formula (I) or pure (3S)-Pyrrolidin-3-ol hydrochloride of formula (II) comprising the steps

a) acid-catalyzed esterification of aminohydroxybutyric acid of formula (IV)

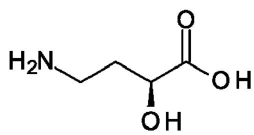

(IV)

with methanol to the methylester hydrochloride of formula (V)

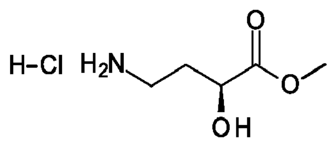

(V)

b) base-induced ring closure reaction of the methyl ester hydrochloride (V) to the lactam of formula (VI)

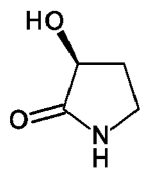

(VI)

c) reducing the amide function in the lactam of formula (VI) to obtain pure (3S)-Pyrrolidin-3-ol hydrochloride of formula (II)

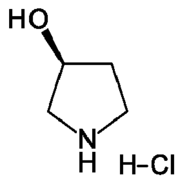

(II)

d) desalination of pure (3S)-Pyrrolidin-3-ol hydrochloride of formula (II) to obtain pure (3S)-Pyrrolidin-3-ol of formula (I)

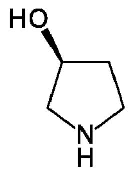

(I)

characterized in that in

in step a) hydrochloride acid is used as acid and the methylester hydrochloride of formula (V) is crystallized from isopropanol and isolated

in step b) sodium phosphate is used as base and the lactam of formula (VI) is crystallized from isopropanol and isolated

in step c) the reduction is performed with lithium aluminum hydride in tetrahydrofuran, followed by purification steps of the crude material when the reaction is complete comprising the substeps

c1) addition of sodium hydroxide solution and water
c2) removal of aluminum salts by filtration and washing the aluminum salts with a mixture of tetrahydrofuran and isopropanol
c3) salt formation and crystallization with hydrochloric acid in isopropanol

in step d) the desalination is performed with an inorganic base whereas the ratio ((3S)-Pyrrolidin-3-ol hydrochloride) to (inorganic base) is more than 1.1 eq and less than 2.5 eq. followed by purification steps of the crude product comprising the substeps:

d1) removal of water by azeotropic distillation using ethanol or isopropanol
d2) filtration to remove inorganic salt
d3) repeating substeps d1) and d2) until the water content is < 0.5 % (m/m) determined by Karl-Fischer titration
d4) addition of methyl tert-butyl ether, 2-methyl-, THF, methylcyclopentyl ether and/or diisopropyl ether as anti-solvent for precipitation of inorganic salts
d5) cooling to -10 to 20 °C and filtration to remove inorganic salts
d6) distillation until (3S)-Pyrrolidin-3-ol is obtained as oil
d7) vacuum distillation of the oil using distillation parameters which allow an overdistillation of (3S)-Pyrrolidin-3-ol.

Another embodiment of the present invention is pure (3S)-Pyrrolidin-3-ol of formula (I)

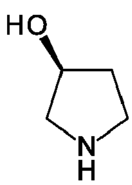

(I)

having

- a chemical purity determined by GC according to method (I) A of not less than 99.80% (area-%)
- an optical purity determined by GC according to method (I) B of not less than 99.9% (enantiomeric excess %ee)
- a content on anhydrous basis determined by potentiometric titration and Karl-Fischer titration according to method (I) C of not less than 99.0%

and containing

- unspecified impurities determined by GC according to method (I) A in an amount less than 0.10% (area-%)
- (S)-Pyrrolidin-3-ol open ring dimer of formula (IX)

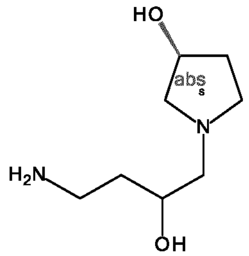

(XI)

determined by GC according to method (I) A in an amount less than 0.05 % (area-%)

- 4-Amino-1,2-butanediol of formula (XII)

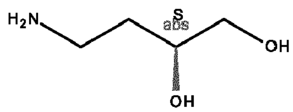

(XII)

determined by GC according to method (I) A in an amount less than 0.05 % (area-%)

Another embodiment of the present invention is pure (3S)-Pyrrolidin-3-ol of formula (I) substantially free of (S)-Pyrrolidin-3-ol open ring dimer of formula (XI).

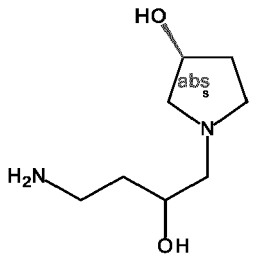

(XI)

determined by GC according to method (I) A.

Another embodiment of the present invention is pure (3S)-Pyrrolidin-3-ol of formula (I) which is substantially free of 4-Amino-1,2-butanediol of formula (XII)

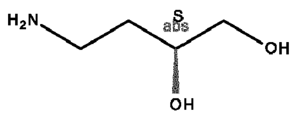

(XII)

determined by GC according to method (I) A.

Another embodiment of the present invention is pure (3S)-Pyrrolidin-3-ol of formula (I) which is substantially free of (S)-Pyrrolidin-3-ol open ring dimer of formula (XI) and of 4-Amino-1 ,2-butanediol of formula (XII).

Another embodiment of the present invention is pure (3S)-Pyrrolidin-3-ol hydrochloride of formula (II)

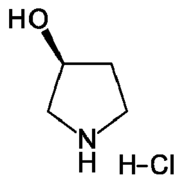
(II)

having

- a chemical purity determined by GC according to method (II) A of not less than 99.5% (area-%)
- an optical purity (enantiomeric excess %ee.) determined by GC according to method (II) B of not less than 99.9% (enantiomeric excess)

Another embodiment of the present invention is a process for preparing pure Larotrectinib of formula (IIIa)

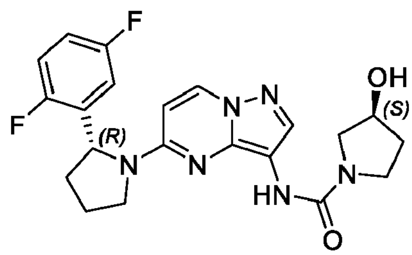
(IIIa)

or a salt thereof,
containing the impurities of formula (XIII) and (XIV)

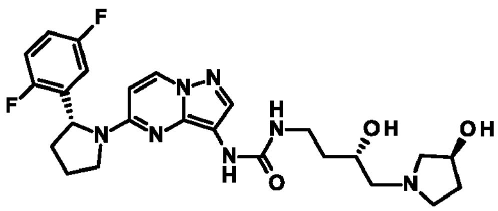
(XIII)

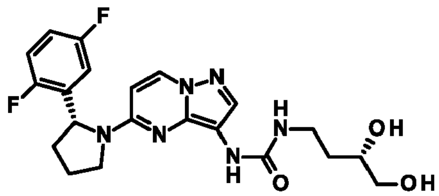
(XIV)

determined by HPLC according to method (III) A each in an amount less than 0.05 % (area%)
by treating a compound of formula 13

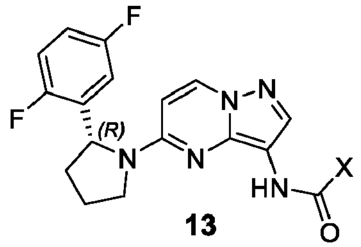

13

or a salt thereof, wherein X is phenoxy with (3S)-Pyrrolidin-3-ol of formula (I)

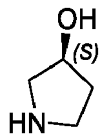

(I)

or a salt thereof to form Larotrectinib or a salt thereof
whereas the compound of formula (I) is obtained by a process for preparing pure (3S)-Pyrrolidin-3-ol of formula (I) or pure (3S)-Pyrrolidin-3-ol hydrochloride of formula (II) according to the present invention.

Another embodiment of the present invention is a process above for preparing a pure sulfate salt of Larotrectinib of formula (IIIb)

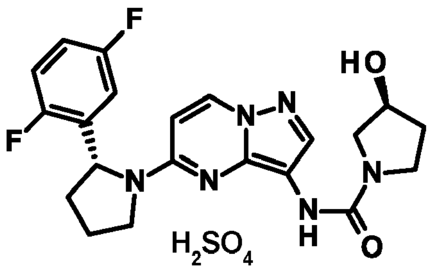

(IIIb)

Another embodiment of the present invention is a process above for preparing pure Larotrectinib of formula (IIIa) or a salt thereof which is substantially free of each of the impurities of formula (XIII) and (XIV) determined by HPLC according to method (III) A.

Another embodiment of the present invention is a process above for preparing a pure sulfate salt of Larotrectinib of formula (IIIb) which is substantially free of each of the impurities of formula (XIII) and (XIV) determined by HPLC according to method (III) A.

Another embodiment of the present invention is pure Larotrectinib of formula (IIIa)

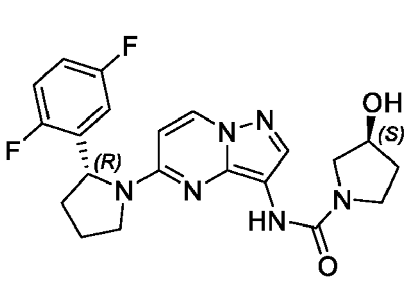

(IIIa)

or a salt thereof,
containing the impurities of formula (XIII) and (XIV)

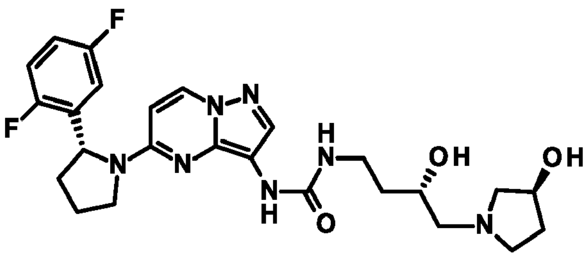

(XIII)

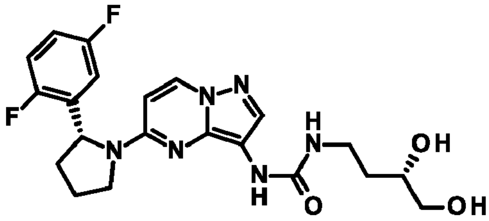

(XIV)

determined by HPLC according to method (III) A each in an amount less than 0.05 % (area-%).

Another embodiment of the present invention is a process above for preparing a sulfate salt of Larotrectinib of formula (IIIb)

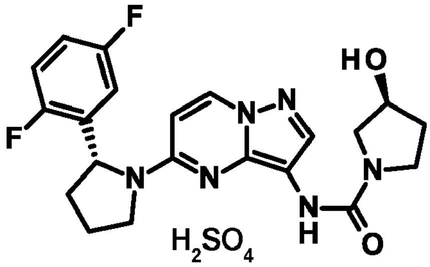

(IIIb)

containing the impurities of formula (XIII) and (XIV)

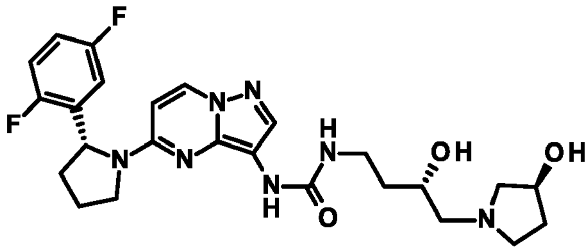

(XIII)

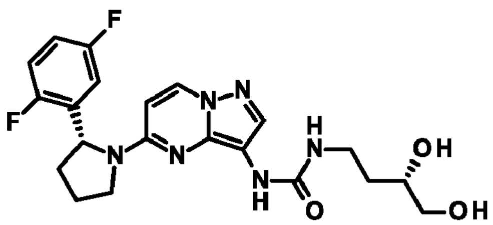

(XIV)

determined by HPLC according to method (III) A each in an amount less than 0.05 % (area-%).

Another embodiments of the present invention are pure Larotrectinib of formula (IIIa) or a salt thereof or a pure sulfate salt of Larotrectinib of formula (IIIb) which are substantially free of each of the impurities of formula (XIII) and (XIV) determined by HPLC according to method (III) A.

Another embodiment of the present invention is the use of pure (3S)-Pyrrolidin-3-ol of formula (I) according to the present invention for preparing Larotrectinib according to formula (IIIa) or a salt thereof

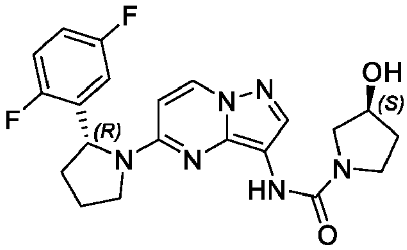

(IIIa)

for a step in which the compound of formula 13

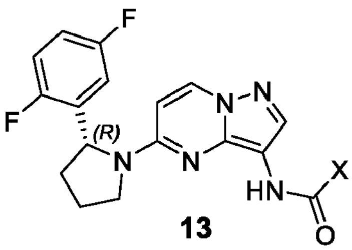

**13**

or a salt thereof, wherein X is phenoxy
is treated with pure (3S)-Pyrrolidin-3-ol of formula (I)

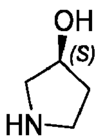

**(I)**

or a salt thereof to form Larotrectinib or a salt thereof.

Another embodiment of the present invention are pharmaceutical compositions comprising pure Larotrectinib of formula (IIIa) or a salt thereof containing the impurities of formula (XIII) and (XIV) determined by HPLC according to method (III) A each in an amount less than 0.05 % (area-%) or which are substantially free of each of the impurities of formula (XIII) and (XIV) determined by HPLC according to method (III) A.

Another embodiment of the present invention is the use of pure (3S)-Pyrrolidin-3-ol of formula (I) according to the present invention or prepared by the process of the present invention for preparing a sulfate salt of Larotrectinib of formula (IIIb)

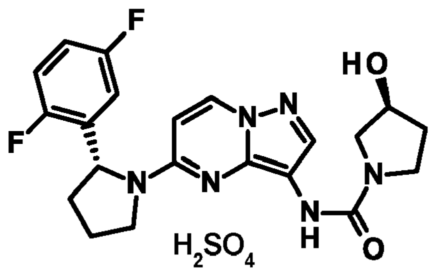

(IIIb)

Examples

Analytical Methods:

Potential by-products not yet listed in the text are provided in table 1.

Table 1:

| Short name | IUPAC-Name | Structural formula |
|---|---|---|
| (S)-AHB acid isopropylester (VIII) | (3S)-3-hydroxy-4-isopropoxy-4-oxo-butylamine | 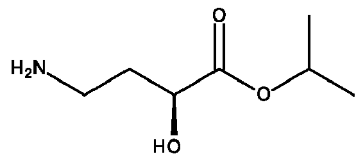 |
| (S)-AHB acid methylester amide (IX) | methyl (2S)-4-acetamido-2-hydroxy-butanoate | 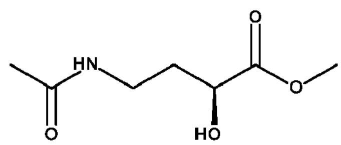 |
| (S)-4-Amino-2-hydroxybutanoic acid Dimer (X) | (2S)-4-[[(2S)-4-amino-2-hydroxy-butanoyl]amino]-2-hydroxy-butanoic acid | 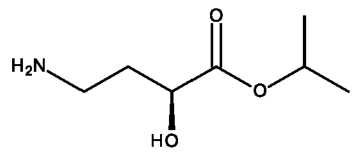 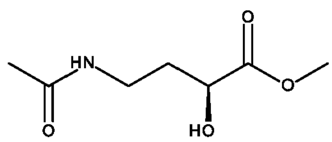 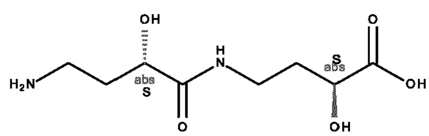 |
| (R)-pyrrolidin-3-ol (XIII) | (R)-pyrrolidin-3-ol | 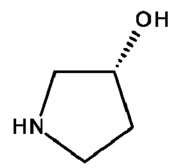 |

**Methyl (2S)-4-amino-2-hydroxy-butanoate hydrochloride (V)**

Two HPLC methods are used to determine content and organic impurities of methyl (2S)-4-amino-2-hydroxy-butanoate hydrochloride (V)

Method (V) A

The HPLC method is used to determine the content of Methyl (2S)-4-amino-2-hydroxybutanoate hydrochloride (V) and the potential by-products (2S)-4-amino-2-hydroxy-butanoic acid (IV) and (S)-AHB acid isopropylester (VIII). For content determination of the main component and the by-products calibration solutions of Methyl (2S)-4-amino-2-hydroxybutanoate hydrochloride (V) are used at 100 % concentration level and 1 % concentration level respectively.

Stationary phase: Acclaim Trinity P1 (150 mm, 3.0 mm ID, 3 μm particle size); mobile phase A: 2300 mg NH4H2PO4 and 310 μL ortho-Phosphoric acid 85% / 1 L water; mobile phase B: acetonitrile; test solution: mobile phase A : acetonitrile 70:30 (v/v); UV detection at 210 nm; oven temperature: 30 °C; injection volume: 5 μL; flow: 1.0 mL/min; isocratic elution: 70 % A : 30 % B (v/v); runtime: 8 minutes; relevant potential by-products: (2S)-4-amino-2-hydroxybutanoic acid (IV) at RRT (relative retention time) 0.75 (RT=1.8 min), (S)-AHB acid isopropylester (VIII) at RRT 0.86 (RT=2.1 min); main component Methyl (2S)-4-amino-2-hydroxy-butanoate hydrochloride (V) RRT 1.00 (RT 2.5 min).

Method (V) B

The HPLC method is used to determine the content of the potential by-products (3S)-3-hydroxypyrrolidin-2-one (VI) and (S)-AHB acid methylester amide (IX). A calibration solution of (3S)-3-hydroxypyrrolidin-2-one (VI) at 1 % concentration level is used for content determination of the by-products.

Stationary phase: YMC Triart C18 (150 mm, 3.0 mm ID, 3 μm particle size); mobile phase A: 1 mL ortho-Phosphoric acid 85% / 1 L water; mobile phase B: acetonitrile; test solution: 10 mM aq. ammonium dihydrogenphosphate; UV detection at 200 nm; oven temperature: 20 °C; injection volume: 5 μL; flow: 0.7 mL/min; linear gradient after 3.5 minutes isocratic run in 2 steps: 0 % B -> 5 % B (0.5 min), 3 minutes holding time at 5 % B, 5 % B -> 30 % B (0.01 min), 5 minutes holding time at 30 % B; relevant potential by-products: (3S)-3-hydroxypyrrolidin-2-one (VI) at RRT (relative retention time) 1.00 (RT=2.0 min) and (S)-AHB acid methylester amide (IX) at RRT 3.29 (RT=6.5 min).

**(3S)-3-hydroxypyrrolidin-2-one (VI)**

Two HPLC methods are used to determine content and organic impurities of (3S)-3-hydroxypyrrolidin-2-one (VI).

Method (VI) A

The HPLC method is used to determine the content of (3S)-3-hydroxypyrrolidin-2-one (VI) and the potential by-product (S)-AHB acid methylester amide (IX). For content determination of the main component and the by-products calibration solutions of (3S)-3-hydroxypyrrolidin-2-one (VI) are used at 100 % concentration level and 1 % concentration level respectively. Stationary phase: YMC Triart C18 (150 mm, 3.0 mm ID, 3 μm particle size); mobile phase A: 1 mL ortho-phosphoric acid 85% / 1 L water; mobile phase B: acetonitrile; test solution: 10 mM aq. ammonium dihydrogenphosphate; UV detection at 225 nm for content determination of (3S)-3-hydroxypyrrolidin-2-one (VI) and at 200 nm for content determination of the relevant potential by-products listed below ; oven temperature: 20 °C; injection volume: 5 μL; flow: 0.7 mL/min; equilibration time prior to start of run: 12 minutes; linear gradient after 3.5 minutes isocratic run in 2 steps: 0 % B -> 5 % B (0.5 min), 3 minutes holding time at 5 % B, 5 % B -> 30 % B (0.01 min), 5 minutes holding time at 30 % B; (3S)-3-hydroxypyrrolidin-2-one (VI) at RRT (relative retention time) 1.00 (RT=2.0 min); relevant potential by-products: (S)-AHB acid methylester amide (IX) at RRT 3.29 (RT=6.5 min).

Method (VI) B

The HPLC method is used to determine the content of the potential by-products methyl (S)-4-amino-2-hydroxybutanoate (V), (S)-4-amino-2-hydroxybutanoic acid dimer (X), (2S)-4-amino-2-hydroxy-butanoic acid (IV) and (S)-AHB acid isopropylester (VIII). A calibration solution of Methyl (2S)-4-amino-2-hydroxy-butanoate hydrochloride (V) at 1 % concentration level is used for content determination of the by-products.

Stationary phase: Acclaim Trinity P1 (150 mm, 3.0 mm ID, 3 μm particle size); mobile phase A: 2300 mg NH4H2PO4 and 310 μL ortho-Phosphoric acid 85% / 1 L water; mobile phase B: acetonitrile; test solution: mobile phase A : acetonitrile 70:30 (v/v); UV detection at 210 nm; oven temperature: 30 °C; injection volume: 5 μL; flow: 1.0 mL/min; isocratic elution: 70 % A : 30 % B (v/v); runtime: 8 minutes; relevant potential by-products: (S)-4-Amino-2-hydroxybutanoic acid Dimer (X) RRT 0.70 (1.7 min), (2S)-4-amino-2-hydroxy-butanoic acid (IV) at RRT (relative retention time) 0.75 (RT=1.8 min), (S)-AHB acid isopropylester (VIII) at RRT 0.86 (RT=2.1 min); Methyl (2S)-4-amino-2-hydroxy-butanoate is set to RRT 1.00 (RT 2.5 min).

**(3S)-pyrrolidin-3-ol (I)**

Method (I) A: Determination of chemical purity

The chemical purity of (3S)-pyrrolidin-3-ol (I) was determined using gas chromatography using

- as detection FID,
- as column an Agilent HP-5 Capillary Column of 15 m length, 0.53 mm inner diameter and 5 μm film thickness
- as column temperature program a program which holds the temperature for 5 minutes at 120°C, raises the temperature with 10 °C/minute from 120°C to 280 C which is thereafter held for 10 minutes,
- as carrier gas helium
- as gas flow a constant flow of 3.0 mL per minute
- as inlet temperature 270 °C
- as detector temperature 280°C
- as diluent methanol and
- an injection volume of 1μl;
- as test solution a solution of 125 mg sample in 5.0 mL of the diluent

Relevant potential by-products are 4-aminobutane-1,2-diol (XII) at RT about 8.5 min and (S)-pyrrolidin-3-ol open ring dimer (XI) at RT about 16.3 min.
The main component (3S)-pyrrolidin-3-ol (I) elutes at RT about 4.4 min.
The following relative response factors (RRF) are used:

- (3S)-pyrrolidin-3-ol (I): 1.00
- 4-aminobutane-1,2-diol (XII): 1.23
- (S)-pyrrolidin-3-ol open ring dimer (XI): 1.00

A relative response factor (RRF) of 1.00 is used for all other impurities.
The largest impurity that is not one of the two specified impurities, i.e. 4-aminobutane-1,2-diol (XII) and (S)-pyrrolidin-3-ol open ring dimer (XI), is reported as "any unspecified impurity".
The content (area-%) of the individual impurities is calculated using the following formula:

$$C_{(i)}\% = \frac{A_{(i)} * RRF_{(i)}}{\sum A_{(i)}} * 100$$

$C_{(i)}$ %: content of component i expressed as area-%
$A_{(i)}$: peak area of component i in the test solution
$RRF_{(i)}$: relative response factor of component i
$\Sigma A_{(i)}$: sum of all peak areas in the test solution

A peak for the residual solvent isopropanol can sometimes be detected on older columns. Isopropanol is separately determined using headspace-GC. Therefore, the peak for isopropanol is not counted in for the GC purity method.
Purity (area-%) of (3S)-pyrrolidin-3-ol (I) is calculated using the formula below:

$$\text{Purity} = 100 - \sum c_{(i)}$$

Purity: purity of (3S)-pyrrolidin-3-ol (I) (area-%)
$\Sigma c_{(i)}$: sum of all organic impurities

Only impurities ≥ 0.05 area-% are included in the sum of all organic impurities.

Method (I) B: Determination of enantiomeric excess %ee

Enantiomeric excess of (3S)-pyrrolidin-3-ol (I) was determined after derivatization using gas chromatography and FID detection:

a) Derivatization of (3S)-pyrrolidin-3-ol (I)

The derivatization according to

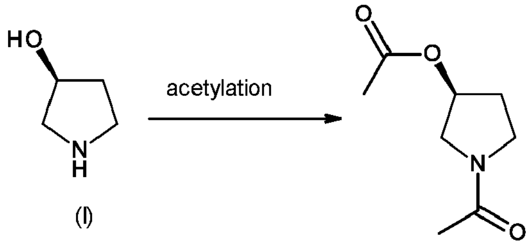


(I)

(VII)

is performed by

1) dissolving 3.00 g of (3S)-pyrrolidin-3-ol (I) in 45 mL of dichloromethane (DCM)
2) adding 7.05 g of triethylamine and 45 mg of N,N-dimethylaminopyridine (DMAP) and cooling the solution in an ice bath and under a nitrogen atmosphere.
3) adding 7.77 g of acetic anhydride dropwise over a period of 5-10 min at <12 °C
4) stirring the solution at room temperature for at least 2 hours and warming up.
5) adding 90 mL water
6) stirring the two-phase mixture for 10 minutes
7) separating the organic phase off and washing with 90 mL water for 10 minutes
8) separating the organic phase off and drying with a few grams of sodium sulfate while stirring,
9) filtering the organic phase and concentrating the filtrate in vacuo.

About 2.25 g of a yellowish oily substance are obtained and the derivatized sample as (3S)-pyrrolidin-3-ol (I) diacetate is transferred for % ee determination by GC.

b) gas chromatography

Gas chromatography of the derivatized sample (3S)-pyrrolidin-3-ol (I) diacetate for the determination of the optical purity is performed using

- as detection FID,
- as column an Agilent CP-Chirasil-DEX CB Chiral Capillary Column of 25 m length, 0.25 mm inner diameter, 0.25 μm film thickness
- as column temperature program a program which holds the temperature for 25 minutes at 120°C, raises the temperature with 3 °C/minute from 120°C to 135 C which is thereafter held for 15 minutes,
- as carrier gas helium
- as gas flow a constant flow of 2.5 mL per minute
- as inlet temperature 220 °C
- as detector temperature 250°C
- as diluent acetonitrile and
- an injection volume of 1μl;
- as test solution a solution of 20 mg of the derivatized sample in 10.0 mL of the diluent

The relative retention times (RRT) of the derivatized (3S)-pyrrolidin-3-ol (I) is at RRT 1.0 and of the derivatized (3R)-pyrrolidin-3-ol (XIII) at about RRT 1.1.

Enantiomeric excess (%ee) is calculated using the following formula:

$$\%ee = \frac{(A_{(S)} - A_{(R)}) * 100}{A_{(S)} + A_{(R)}}$$

%ee: chiral purity of (3S)-pyrrolidin-3-ol (I) as % enantiomeric excess

$A_{(S)}$: peak area of derivatized (3S)-pyrrolidin-3-ol (I) in the test solution
$A_{(R)}$: peak area of derivatized (3R)-pyrrolidin-3-ol (XIII) in the test solution
100: factor needed for conversion to %

Method (I) C: Determination of content on anhydrous basis

The content is calculated on anhydrous basis (content (i.d.s.)) using potentiometric titration and determination of the water content by Karl-Fischer titration.

*Potentiometric titration:*

The content of (3S)-pyrrolidin-3-ol (I) was determined using

- as apparatus a titrator, e.g. Metrohm Titrando 905 equipped with sample changer 815
- as electrode a combination electrode for anhydrous titrations
- as test solution a solution of 150-250 mg sample accurately weighed in 100 mL of acetic acid
- as titrant a 0.1 mol/L perchloric acid
- using the following formula for content

$$\text{Content (\%)} = \frac{V * t * 87{,}12 * 100 * F}{E}$$

V: volume of titrant used (mL)
t: titer of the 0.1 mol/L perchloric acid
F: factor of the perchloric acid, i.e. the nominal concentration (0.1 mol/L)
E: sample weight (mg)
87.12: molar mass of (3S)-pyrrolidin-3-ol (I) (mg/mmol)
100: factor needed for conversion to %

*Karl-Fischer titration:*

The water content of (3S)-pyrrolidin-3-ol (I) was determined using

- as apparatus a KF-coulometer
- as titration medium Coulomat AG or comparable commercially available reagent for coulometric determination and with addition of Coulomat CG
- as extraction time 180 s
- as sample weight 400-600 mg accurately weighed

Calculation of content (i.d.s.) (% (m/m)):

The content calculated on anhydrous basis ((content (i.d.s.)) of (3S)-pyrrolidin-3-ol (I) was calculated using the following formula:

$$\text{Content (i.d.s)(\%)}: = \frac{\text{content} * 100}{100\text{-}A}$$

Content (i.d.s)(%): content calculated on anhydrous basis of (3S)-pyrrolidin-3-ol (I) (% (m/m))
content: content of (3S)-pyrrolidin-3-ol (I) determined using potentiometric titration (% (m/m))
100: factor needed for conversion to %
A: nominal value of water content of (3S)-pyrrolidin-3-ol (I) determined using Karl-Fischer titration (% (m/m))

**(3S)-pyrrolidin-3-ol hydrochloride (II)**

Method (II) A: Determination of chemical purity

The chemical purity of (3S)-pyrrolidin-3-ol hydrochloride (II) was determined using gas chromatography using

- as detection FID,
- as column an Agilent HP-5 Capillary Column of 15 m length, 0.53 mm inner diameter and 5 μm film thickness,
- as column temperature program a program which holds the temperature for 5 minutes at 120°C, raises the temperature with 10 °C/minute from 120°C to 280 C which is thereafter held for 10 minutes,
- as carrier gas helium
- as gas flow a constant flow of 3.0 mL per minute
- as inlet temperature 270 °C
- as detector temperature 280°C
- an injection volume of 1μl;
- as test solution a solution of 25 mg sample in 1.0 mL isopropanol / water (V/V 8/2) and 30 μl of aqueous sodium hydroxide (about 25 % (m/m)); the test solution is performed in the injection vial and the sample solution is homogenized by ultrasonification (3-5 minutes)
- as injection sequence a sequence that is characterized by including at least two blank injections of the diluent (1.0 mL isopropanol / water (V/V 8/2) and 30 μl of aqueous sodium hydroxide (about 25 % (m/m))) between injections of the control solution and the sample solution as well as between injections of the sample solution

Relevant potential by-products are 4-aminobutane-1,2-diol (XII) at RT about 8.5 min and (S)-pyrrolidin-3-ol open ring dimer (XI) at RT about 16.3 min.
The main component (3S)-pyrrolidin-3-ol (I) elutes at RT about 4.4 min.
The following relative response factors are used:

- (3S)-pyrrolidin-3-ol (I): 1.00
- 4-aminobutane-1,2-diol (XII): 1.23
- (S)-pyrrolidin-3-ol open ring dimer (XI): 1.00

A relative response factor (RRF) of 1.00 is used for all other impurities.

Method (II) B: Determination of optical purity:

The optical purity of (3S)-pyrrolidin-3-ol hydrochloride (II) is determined according to method (I) B after (3S)-pyrrolidin-3-ol hydrochloride (II) was converted to (3S)-pyrrolidin-3-ol (I): Converting the (3S)-pyrrolidin-3-ol hydrochloride (II) to (3S)-pyrrolidin-3-ol (I):
The conversion of (3S)-pyrrolidin-3-ol hydrochloride (II) to (3S)-pyrrolidin-3-ol (I) is performed by

1) placing at room temperature 40 g of (3S)-pyrrolidin-3-ol hydrochloride (II) in 314 mL of isopropanol
2) adding 16 mL of deionized water.
3) adding 54.8 mL of 25% aqueous sodium hydroxide solution to obtain a pH of approx. pH 13, after 5 min
4) stirring for two hours at room temperature
5) azeotropic distillation of the isopropanol / water mixture at internal temperature 82 °C
6) repeating 5) several times with 314 mL of isopropanol until a water value (KF, Karl Fischer) of <0.5% (m/m) is reached and a final volume of approx. 300 ml
7) adding 200 mL of isopropanol and suctioning the precipitated salts off through a frit coated with kieselgur
8) washing the salts with 60 mL of isopropanol
9) concentrating the filtrate to an oil at approx. 50 °C/20 mbar and
10) purification of the remaining oil by short-path distillation with a bath temperature of 125-130 °C and a pressure of approx. 3-4 mbar

For method (I) B a portion of 3.00 g of (3S)-pyrrolidin-3-ol (I) is derivatized to form (3S)-pyrrolidin-3-ol (I) diacetate (VII).
Table 2 lists the starting material, intermediates and end products of the process of the present invention:

Table 2:

| Structure | IUPAC-Name |
|---|---|
| 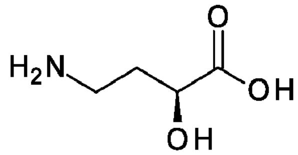 Formula Weight: 119,119(3) Formula : $C_4H_9NO_3$ | (2S)-4-amino-2-hydroxy-butanoic acid (IV) |
| 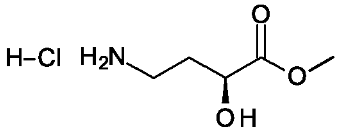 Formula Weight: 169,607(5) Formula : $C_5H_{11}NO_3.ClH$ | methyl (2S)-4-amino-2-hydroxybutanoate hydrochloride (V) |
| 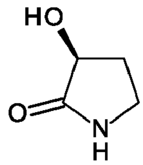 Formula Weight: 101,104(3) Formula : $C_4H_7NO_2$ | (3S)-3-hydroxypyrrolidin-2-one (VI) |
| 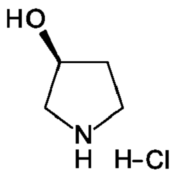 Formula Weight: 123,581 (4) Formula : $C_4H_9NO.ClH$ | (3S)-pyrrolidin-3-ol hydrochloride (II) |
| 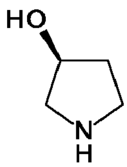 Formula Weight: 87,120(3) Formula : $C_4H_9NO$ | (3S)-pyrrolidin-3-ol (I) |

**Example 1**

**Example 1a: Methyl (2S)-4-amino-2-hydroxy-butanoate hydrochloride (V)**

350.0 g (2.938 mol) of (2S)-4-amino-2-hydroxy-butanoic acid (IV) were placed in 3500 mL of MeOH (suspension) at room temperature in a double jacket glass reactor (impeller stirrer 200 rpm) and was heated to an internal temperature of 62 °C (jacket temperature of 70 °C) within 30 minutes. At this temperature, 461.3 g (5.876 mol) of acetyl chloride were added via a dropping funnel over the course of 2 hours 30 minutes. After 2 hours 30 minutes the inner temperature was 60.1 °C and the jacket temperature 68 °C, it was an almost clear solution, slow evolution of gas under reflux. Methanol was removed by distillation until a volume of 1.4 L was reached. Then 1400 mL of methanol was added and again was removed by distillation to a volume of 1.4 L. It was cooled to an internal temperature of 5 °C (jacket temperature was 2 °C) which resulted into a yellow somewhat viscous solution.

Then 2800 mL of isopropyl alcohol are poured in, the internal temperature rises to 13.5 °C, a few crystals were already formed, it completely crystallized after 1 minute! The internal temperature rises to 18.5 °C. At an internal temperature of 16-26 °C and a jacket temperature of 50-55 °C., the solvent was distilled off under reduced pressure to a residual volume of about 1800 mL, a white suspension that was still easily stirrable was obtained. It was cooled down to an internal temperature of -10 °C within one hour and stirred for 1.0 hours at this temperature. Crystals (suspension 1700 mL = 1539.7g) were isolated over a 1000 mL P3 frit (duration: 2.5 minutes) and then was washed with 200 g cold isopropyl alcohol (duration: <1 minute), the frit is filled with approx. 800 mL of moist product.

Moist yield: 568.5 g
It was dried under vacuum at 40 °C under nitrogen at <100 mbar approximately overnight.
Yield: 457.7 g (91.8 % th.) of white crystalline material.
Residual solvent: 0.096 % (m/m) MeOH and 0.591 % (m/m) isopropyl alcohol
20.55% by weight chloride (theory: 21.5% by weight = mono-hydrochloride)
Mass: $[M+H^+] = 134$
$^1$H-NMR (600 MHz, DMSO-$d_6$): $\delta$ = 1.85 (m, 1H), 2.05 (m, 1H), 2.89 (s, 2H), 3.18 (s, 3H), 4.23 (s, 1H), 5.80 (s, 1H), 8.25 (s, 3H).

**Example 1b: (3S)-3-hydroxypyrrolidin-2-one (VI)**

In a double jacket glass reactor 250.0 g (1.474 mol) Methyl (2S)-4-amino-2-hydroxybutanoate hydrochloride (V) were dissolved in 1500 mL methanol at room temperature. The internal temperature dropped to 18 °C, it was dissolved very quickly, a clear yellow solution was obtained. This solution was filtered (over a P3 frit which was covered with approx. 1 cm of diatomite and moistened with methanol) and the filter was washed with 70 g of methanol. A yellow clear solution: 1488.4 g = 1700 mL was obtained. Then 277.9 g (1.695 mol) of trisodium phosphate were placed in a double jacket glass reactor (with an impeller stirrer) and 1300 mL of methanol were added and was heated to an internal temperature of 40 °C and a jacket temperature of 41 °C. At an internal temperature of 40 °C, the 1700 mL methanolic solution of the methylester hydrochloride (V) was added in over the course of 4.5 hours (target: 5 hours) using a dropping funnel and rinsed with 15 g of methanol. Then it was heated to an internal temperature of 50 °C (jacket temperature = 52 °C) and was stirred for 0.75 hours at this temperature. At an internal temperature of 50 °C 554 g of deionized water was added in within 10 minutes (the suspension is very stirrable). It was stirred for 35 minutes at an internal temperature of 50 °C. Then the salts were removed by filtration (1L P3 frit, duration: ~ 2 minutes) and the salt cake washed three times with 150 mL of warm methanol. The salts which were filtered had a moist weight of 356.5 g. In this way a filtrate (light yellow and clear) of 3206.7 g = 3780 mL was obtained. The solution was transferred to the 4L double jacket glass reactor and the solvent was removed by vacuum distillation at an internal temperature of 25 °C to 32 °C and a jacket temperature of 30 to 60 °C to a residual volume of about 500 mL.

Then 2500 mL of isopropyl alcohol were added and immediate formation of a thick white suspension, but easily stirrable was observed. Again, the solvent was removed by distillation under vacuum at an internal temperature of 26 °C to 32 °C and a jacket temperature of 50 to 60 °C to a residual volume of about 500 ml. The residue of about 500 mL was mixed with 2500 mL of isopropyl alcohol und the water content was measured by Karl-Fischer titration (KF = 3.43% (m/m)). It was heated to an internal temperature of 50 °C and was stirred for one hour at an internal temperature of 50 °C. The warm suspension is filtered through a 1000 mL P3 frit (duration: 6 minutes, approx. 1 cm thick layer of salt), and was washed twice with 250 mL of warm isopropyl alcohol each. 2491.8 g= 3150 mL of a light yellow and clear filtrate was obtained. At this point the water content was measured: KF = 3.02% (m/m). The lightyellow clear solution was concentrated on a rotary evaporator to a residual volume of about 900 mL (741.9 g) and was transferred to the 1 L double jacket glass reactor and rinsed with 50 mL of isopropyl alcohol. The solvent was removed by distillation under vacuum (inner temperature 30 °C and jacket temperature 50 °C) to a residual volume of approx. 400-450 ml, the product crystallized out during the process. The distillation was stopped, and the mixture was stirred for about 1 hour at an internal temperature of 45 °C. The caking above the liquid level dissolved and a white, easily stirrable suspension was obtained. The suspension was cooled to an internal temperature of 22 °C within 2.0 hours, during which more crystallization was observed. Stirring at an internal temperature of 22 °C was performed overnight (approx. 14 hours). Then it was cooled to an internal temperature of -10 °C within two hours, then a thick, easily stirrable suspension was obtained. The suspension was subsequently stirred at an internal temperature of -10 °C for 2.5 hours. Crystals were isolated by filtration (suspension: 400 mL = 367.5 g) via a 250 mL P3 frit (~ 85% filled), the reactor rinsed once with the mother liquor and was washed with 50 mL of cold isopropyl alcohol.

Wet yield: 170.6 g
The product was dried under vacuum at 35 °C (under nitrogen auxiliary air at <100 mbar approximately overnight).
Yield: 124.9 g (83.81 % th.) of white crystals

Residual solvent: 0.00 % (m/m) MeOH and 1.01 % (m/m) isopropyl alcohol
Mass: $[M+H^+] = 102$
$^1$H-NMR (400 MHz, DMSO-$d_6$): δ = 2.23 (m, 1H), 2.25 (m, 1H), 3.02-3.19 (m, 2H), 3.96 (t, 1H), 5.02-6.01 (s, 1H), 6.99-7.98 (s, 1H).

**Example 1c: Pure (3S)-pyrrolidin-3-ol hydrochloride (II)**

In a double jacket glass reactor 120 g (1.187 mol) (3S)-3-hydroxypyrrolidin-2-one (VI) were suspended in 1300 mL of THF (tetrahydrofuran) and then heated to 25 °C jacket temperature. At this temperature was added a solution of 1582.5 mL 2.4 molar lithium aluminum hydride (LAH) solution in THF within 45 min.. During the addition (~ 190 ml) a strong evolution of gas was observed. Inner temperature increased from 25 °C to 32 °C temperature, after approx. 110 mL addition, the temperature slowly fell to 28 °C (after the first 270 mL were added no gas evolution was observed). It was then heated to 68 °C inner temperature and then stirred for 48 hours at this temperature.

After 48 hours the batch was cooled to -3 °C (inner temperature), then a mixture of 144 mL of deionized water and 432 mL of THF was slowly added (over 100 min), the temperature increased to 19 °C with strong gas evolution. A white suspension was obtained by the end of addition but was still stirrable. It was then cooled to 0 °C (inner temperature). At this temperature (0°C) a solution of 144 mL of 15% aq. NaOH was added (over 25 min) temperature increase to 21 °C (slight gas evolution, jacket temperature -5 °C, well stirrable suspension). It was cooled to 0 °C, and then 432 mL deionised water were added (in 15 minutes, very slight gas evolution, jacket temperature -5 °C, suspension). The mixture was stirred at 0-3 °C inner temperature for 10 min. and then the batch was heated to 68 °C inner temperature and 1000 mL of THF were distilled off. Then 1100 mL of isopropyl alcohol were added. The batch was stirred for 2 h at 68 °C inner temperature. It was then cooled to 50 °C inner temperature. At this temperature the salts (aluminum salts) were filtered off (in approx. 10 min) and the salt cake was washed two times with each 440 mL of a warm THF / isopropyl alcohol 1: 1 mixture. (in approx. 5 min). The THF / isopropyl alcohol solution was then concentrated at 50 °C and 150 mbar by distillation to 312.4 g solution. Water value (KF): 14.92%. Then 1090 mL of isopropyl alcohol was added to the 312.4 g remaining solution and it was again concentrated at 50 °C and 150 mbar by distillation to a residual amount of 304.1 g (Water value KF: 2.52%). 544 mL of 2-propanol were added to the remaining amount of 304.1 g and it was again concentrated at 50 °C and 150 mbar by distillation to a residual volume of 285.71 g (Water value KF: 0.06%). Then 400 mL of MTBE were added, and the mixture was cooled to 0-5 °C inner temperature. At 0-5 °C 273 mL of 5-6 N hydrochloric acid in 2-propanol were added within 60 minutes. Temperature increased to 8 °C, thick yellowish suspension was obtained. An additional 338 mL of methyl tert. butyl ether (MTBE) were added, then the inner temperature was raised to 60 °C, it was stirred at this temperature for 1 h. The batch was cooled to 2 °C inner temperature and was stirred for 2 hours. The product was isolated by filtration. It was washed two times with each 50 mL a mixture of cold MTBE / isopropyl alcohol 4: 1. The solid was dried under vacuum overnight at 50 °C and 20 mbar.

The dried solid was dissolved in 404.8 mL of isopropyl alcohol and then 22.1 mL of 5-6 N HCl in 2-propanol were added and was heated to 82 °C inner temperature and then stirred for 1h at this temperature. Then it was cooled to 50 °C inner temperature. Then 633.6 mL of MTBE were added in 60 min. After the addition was completed, it was heated to 62 °C inner temperature and it was stirred for 1 hour at this temperature. The batch was cooled to 2 °C inner temperature (in 2 hours), then stirred for 1 hour at 2 °C, the product was filtered off and was washed two times with each 110 mL of a cold mixture (2 °C) of isopropyl alcohol / MTBE 1: 1.5. The solid was dried under vacuum overnight at 50 °C and 20 mbar.

Yield: 105.02 g (71.6 % th.) of crystalline material with off-white color.
Enantiomeric excess: > 99.9 % (99.95% ee)
29.2% (m/m) chloride
Mass: $[M+H^+] = 88$
$^1$H-NMR (400 MHz, DMSO-$d_6$): δ = 1.85 (m, 2H), 3.00 (m, 1H), 3.09-3.21 (m, 3H), 4.39 (s, 1H), 5.39 (s, 1H), 9.41 (s, 2H).

**Example 1d: Pure (3S)-pyrrolidin-3-ol (I)**

100 g (809.17 mmol) (3S)-pyrrolidin-3-ol hydrochloride (II) (with 99.96 %ee) were suspended in 593 g of methanol (750 ml). Then 116.5 g of 25% sodium hydroxide solution (0.9 eq.) were added over 15 minutes and the mixture was subsequently stirred at 20°C for 30 minutes (pH 11-12 at this point). To this suspension was then added 132.7 g (809.17 mmol) of trisodium phosphate and the mixture was subsequently stirred at 50 °C inner temperature for 2 h. A thick, yellow suspension, (pH 11-12) was obtained. After 2 h 73 g of deionized water were added at 50 °C and the batch was stirred at 50 °C inner temperature for 1 hour. The suspension was cooled to 20°C, stirred for 1 h at 20°C and the inorganic salts filtered off through a 0.5 L P3 frit. The flask and the solid were washed with the filtrate and then twice

with each 59 g isopropyl alcohol (75 ml) afterwards. A yellow solution (1 L / 825 g) was obtained (water content KF: 17.9% (m/m)). Then approx. 800 mL of solvent were removed by distillation at approx. 70-90 °C inner temperature, 90-110 °C jacket temperature (duration approx. 3 h). To the residue were added 628 g of isopropyl alcohol (800 ml) and again solvent was distilled off to approx. 660 mL residual volume (water content KF: 9.8% (m/m)). The residue was concentrated to a residual volume of 200 mL (amount of distillate approx. 800 mL / 650 g, duration approx. 2 h). To the residue was added 628 g of isopropyl alcohol (800 ml) and again 330 mL of solvent was distilled off (approx. 660 mL residual volume, water content KF: 1.6% (m/m)). The residue was concentrated to a residual volume of 200 mL (amount of distillate approx. 800 mL / 635 g, duration approx. 2 h). To this residue again 628 g of isopropyl alcohol (800 ml) are added (yellow suspension) and again 330 mL of solvent was distilled off (duration approx. 1 h, approx. 660 residual volume, water content KF: 0.2% (m/m)). The yellow suspension was cooled to 50 °C, then 246 g tert-butyl methyl ether (MTBE) (333 ml) were added, the mixture was stirred for 10 minutes and cooled to 0-5 °C. The batch is stirred for 2 h at 0-5 °C. The inorganic salts were filtered off (0.5 L P3 frit), and the filtered solid were washed 2 x each 76.5 g of cold isopropyl alcohol / MTBE 2: 1 (100 ml). A yellow solution, 1.1 L / 817 g was obtained. The filtrate is completely evaporated under vacuum (end conditions: 1 h at 65 °C jacket temperature and <25 mbar). After complete solvent removal 66.1 g (I) of a yellow-orange oil was obtained. The oil was distilled (by batch distillation) at 2-3 mbar und approx. 120°C (jacket temperature approx. 150°-160°C)

Yield: 62.50 g (88.7 % th.) of a colorless oil, which crystallizes on standing to a massive crystalline block.
ee of 99.97 %ee
Mass: $[M+H^+] = 88$
$^1$H-NMR (400 MHz, DMSO-$d_6$): $\delta$ = 1.55-1.62 (m, 1H), 1.70-1.78 (m, 1H), 2.63-2.95 (m, 4H), 4.19 (s, 1H), 4.70 (s, 2H).

**Example 1e: Thin layer distillation of crude (3S)-pyrrolidin-3-ol (I)**

Starting with 350.0 g of (3S)-pyrrolidin-3-ol hydrochloride (II) (99.95 %ee) which was prepared according to example 1c, 253.4 g of a crude-(3S)-pyrrolidin-3-ol (I) was obtained after desalination. This crude product was distilled with a thin layer distillation device (short way distillation).

Yield: 224.77 g (91.1 % th.) of a colorless oil, which crystallizes on standing to a massive crystalline block.
ee of 99.96 %ee
Mass: $[M+H^+] = 88$
$^1$H-NMR (400 MHz, DMSO-$d_6$): $\delta$ = 1.54-1.63 (m, 1H), 1.71-1.78 (m, 1H), 2.62-2.95 (m, 4H), 4.20 (s, 1H), 4.71 (s, 2H).

**Example 2**

In example 2 a description of the batch records is presented, how the synthesis of the free base (I) is performed in the pilot plant (batch records are recipes and are written in present form!). According to these batch records the free base (I) is currently prepared on commercial scale.

The quantities are specified for a typical batch size and can be adapted to the size of the apparatus, provided the stoichiometric ratio of the reaction components remains constant. Acceptable ranges derived from process development studies are given in parenthesis. These are the preferred and especially preferred values. The process was validated on production scale.

**Example 2a: Methyl (2S)-4-amino-2-hydroxy-butanoate hydrochloride (V)**

Due to limited reactor capacity the batch was divided. In principle it can be performed in one batch.

The reaction was separately performed for two batches and they were subsequently combined for work-up

Batch 1: A vessel fitted with a stirrer is charged with 395.0 kg (355.5 - 474.0 kg) methanol as the solvent and 50.0 kg (49.6-50.4 kg) (2S)-4-amino-2-hydroxy-butanoic acid is added. The mixture is heated to ≥ 60 °C and 68.0 kg (61.2-74.8 kg) acetyl chloride is added at ≥ 55 °C (50-65°C). Methanol is distilled off (max. jacket T = 100°C). 157.9 kg Methanol is added and again distilled off. The solution is transferred to another vessel.

Batch 2: A vessel fitted with a stirrer is charged with 395.0 kg (355.5-474.0 kg) methanol as the solvent and 50.0 kg (49.6-50.4 kg) (2S)-4-amino-2-hydroxy-butanoic acid is added. The mixture is heated to ≥ 60 °C and 68.0 kg (61.2-74.8 kg) acetyl chloride is added at ≥ 55 °C (50-65°C). Methanol is distilled off (max. jacket T = 100°C). 157.9 kg Methanol is added and again distilled off. The solution is transferred to another vessel and combined with the first batch. To the combined batches is added 624.0 kg (499.2-748.8 kg) isopropyl alcohol and solvent is distilled of at ≤ 30°C and at reduced pressure (≤ 70mbar). It is stirred at -10°C (-13°C to -5°C), the solid is isolated by filtration and washed with 57.1 kg isopropyl alcohol, then dried under reduced pressure at 50°C maximum jacket temperature up to 48 hours.

A yield of 81.7 to 102.7 % th. was obtained, this corresponds to an average yield of 92.0 % th. (related to (2S)-4-amino-2-hydroxy-butanoic acid) of Methyl (2S)-4-amino-2-hydroxybutanoate hydrochloride (V).

**Example 2b: (3S)-3-hydroxypyrrolidin-2-one (VI)**

A vessel fitted with a stirrer is charged with 474.0 kg methanol as the solvent and 100.0 kg (99.0-101.0 kg) methyl (2S)-4-amino-2-hydroxy-butanoate hydrochloride intermediate is added. The mixture is heated to 40 °C (37-43°C). The solution is added over 5 h (2.5 to 9.4 h) to another vessel with 111.2 kg (110.2-112.2 kg) trisodium phosphate in 410.8 kg methanol at 40 °C (20-53°C). It is warmed up and stirred at 50°C (20-53°C). 221.6 kg water is added, and the mixture is stirred at 50°C (20-53°C). The salts are collected by filtration and are washed three times with each 100.0 kg methanol. Filtrate is concentrated by distillation at reduced pressure and a jacket temperature at max. 60°C. 780.0 kg isopropyl alcohol is added and distilled off at reduced pressure and a jacket temperature at max. 60°C. Addition and distillation of additional 780.0 kg isopropyl alcohol is repeated until IPC for water content is ≤ 2% (m/m) (Karl Fischer). The reactor content is warmed up to 50°C (47-53°C) and salts are collected by filtration and washed twice with each 78.0 kg isopropyl alcohol. Filtrate is concentrated by distillation at reduced pressure. It is cooled down to -10°C (-13 to -5°C). The solid is isolated via centrifuge and washed twice with each 15.6 kg isopropyl alcohol. The solid is dried under reduced pressure at max. 50°C jacket temperature for max.72 h.
A yield of 61.6 to 82.4 % th. was obtained, this corresponds to an average yield of 74.5 % th. (related to Methyl (2S)-4-amino-2-hydroxy-butanoate hydrochloride (V)) of (3S)-3-hydroxypyrrolidin-2-one (VI).

**Example 2c: Pure (3S)-pyrrolidin-3-ol hydrochloride (II)**

Due to limited reactor capacity 4 smaller batches were performed and combined for work-up. In principle it can be performed in one batch. In total 66.0 kg of (3S)-3-hydroxypyrrolidin-2-one (VI) is converted.
A vessel fitted with a stirrer is charged with 150.0 kg tetrahydrofuran (THF) as the solvent and 16.5kg (16.3-16.7 kg) (3S)-3-hydroxypyrrolidin-2-one (VI) is added at 30 °C (-10-40°C). 194.0 kg (181.9 - 212.2kg) Lithium aluminum-hydride, 2.4 M in THF is added at 30 °C (-10-40°C) and then it is stirred under reflux at max. jacket temperature of 78°C (max. 72 h). A mixture of 52.8 kg tetrahydrofuran and 19.8 kg water is added at 30°C (0-35°C) and then stirred, 23.0 kg (22.8-23.2 kg) of a 15% aqueous solution of sodium hydroxide is added and stirred at 30°C (0-35°C). 59.4 kg water is added and stirred at 30°C (0-35°C). Solvent is distilled off at a jacket temperature of max. 98°C. 119.3 kg isopropyl alcohol is added, and solvent is distilled off. The reaction mixture is stirred at 68°C (65-71°C) then cooled to 50°C (47-53°C). Salts are collected by filtration and are washed with twice with a 1:1 mixture each of 50.0 kg of tetrahydrofuran and isopropanol. The combined filtrates are distilled at reduced pressure with a jacket temperature of max. 73°C. 468.0 kg isopropyl alcohol is added and distilled off at reduced pressure. This is performed several times until IPC for water content is ≤ 0.5 % (m/m) (Karl Fischer). 168.7 kg methyl tert. butyl ether is added, and the temperature is cooled to 10°C (0-25°C). Then a solution of 34.3 kg (33.8-35.8 kg) HCl in isopropyl alcohol (prepared by dosing HCl gas into isopropyl alcohol at 15°C (5-25°C), approx. 5-6 N) is added at 10°C (0-25°C). Then 143.0 kg methyl tert. butyl ether is added, and it is stirred at 60°C (57-63°C). It is cooled and stirred at 3°C (-10 to 6°C). Solid is isolated by filtration and washed twice with a 4:1 mixture of methyl tert. butyl ether and isopropyl alcohol each 8.9 kg. The solid is dissolved in 257.4 kg isopropyl alcohol at 65°C (62-68°C) and part of isopropyl alcohol is distilled off at reduced pressure with a jacket temperature of max. 73°C. 11.8 kg (11.6.-12.0 kg) of a solution of 5-6 N HCl in isopropyl alcohol (prepared from HCl gas and isopropyl alcohol) is added and stirred at 60°C (57-63°C). 302.8 kg methyl tert. butyl ether is added at 50°C (47-53°C) and stirred at 60°C (57-63°C). It is cooled and stirred at 3°C (0-6°C). The solid is isolated by filtration and is washed twice with a mixture of 1.5:1 each 17.2 kg methyl tert. butyl ether and isopropyl alcohol. The solid is dried under reduced pressure at max. T=50°C at least for 72h. An IPC for optical purity is performed (enantiomeric excess ≥ 99.9 %ee). Batches which do not comply to IPC limit can be reprocessed by dissolving in isopropyl alcohol and by crystallization as described.
A yield of 71.8 to 79.6 % th. was obtained, this corresponds to an average yield of 75.3 % th. (related to (3S)-3-hydroxypyrrolidin-2-one (VI)) of (3S)-pyrrolidin-3-ol hydrochloride (II).

**Example 2d: Pure (3S)-Pyrrolidin-3-ol (I)**

A vessel fitted with a stirrer is charged with 118.6 kg methanol as the solvent and 20.0 kg (19.8-20.2 kg) (S)-Pyrrolidin-3-ol hydrochloride (II). 23.3 kg (12.7-13.6 kg) aqueous 25% sodium hydroxide are added and the mixture is stirred at 25°C (15-30°C). 26.5 kg (21.2 - 31.8kg) trisodium phosphate is added and stirred at 50°C (40-55°C). 14.6 kg water is added and stirred at 50°C (40-55°C). It is cooled to 20°C (15-25°C) and the salts are collected by filtration and washed twice with each 11.8 kg isopropyl alcohol. The filtrate is concentrated by distillation under reduced pressure with a jacket temperature of max. 98°C. 125.6 kg isopropyl alcohol is added and distilled off several times, until IPC for water content < 0.5 % (m/m) (Karl Fischer). 49.2 kg methyl tert. butyl ether is added, and it is stirred at 50°C (47-53°C).

Then it is cooled to 0°C (-3 to 3°C). Salts are collected by filtration and washed with a 2:1 mixture of each 15.3 kg isopropyl alcohol and methyl tert. butyl ether. Filtrate is concentrated by distillation under reduced pressure with jacket temperature of max. 63°C. Product is isolated by short path distillation under reduced pressure with a jacket temperature of max. 120°C. The distillation sump is redistilled using the same parameters to yield a second portion of (3S)-pyrrolidin-3-ol (I).

A yield of 80.1 to 88.7 % th. was obtained, this corresponds to an average yield of 84.4 % th. (related to (S)-Pyrrolidin-3-ol hydrochloride (II)) of (3S)-pyrrolidin-3-ol (I).

**Example 3**

Results of first pilot plant (commercial production) campaign (yields and analytical results)

**Example 3a: Methyl (2S)-4-amino-2-hydroxy-butanoate hydrochloride (V)**

Table 3 shows the yield of the first commercial production campaign of Methyl (2S)-4-amino-2-hydroxy-butanoate hydrochloride (V), started from 2 times 50 kg= 100 kg (2S)-4-amino-2-hydroxy-butanoic acid, 395.0 kg methanol and 68.0 kg acetyl chloride.

Table 3:

| Batch | Yield [kg] | Yield [% th.] |
|---|---|---|
| 1 | 116.3 | 81.7 (first batch)* |
| 2 | 138.1 | 97.0 |
| 3 | 116.4 | 81.7 |
| 4 | 132.4 | 93.0 |
| 5 | 132.6 | 93.1 |
| 6 | 131.4 | 92.3 |
| 7 | 132.3 | 92.9 |
| 8 | 133.4 | 93.7 |
| 9 | 146.2 | 102.7 (last batch)* |
| * there is always some compound on the filter, which is taken into the next batch, which is isolated in the last batch. | | |

A yield of 81.7 to 102.7 % th. was obtained, this corresponds to an average yield of 92.0 % th..

Tables 4 and 5 show the analytical results of these batches.

Table 4:

| **Batch** | **1** | **2** | **3** | **4** | **5** |
|---|---|---|---|---|---|
| Identity (HPLC) | complies | complies | complies | complies | complies |
| Content [% (m/m)] | 97.3 | 97.5 | 98.2 | 98.2 | 97.9 |
| Method (V) A | | | | | |
| (2S)-4-amino-2-hydroxy-butanoic acid (IV) [% (m/m)] | 1.2 | 1.6 | 1.0 | 0.9 | 1.0 |
| (S)-AHB acid isopropylester (VIII) [% (m/m)] | 0.08 | 0.10 | 0.08 | 0.14 | 0.13 |
| | | | | | |
| Method (V) B | | | | | |
| (3S)-3-hydroxypyrrolidin-2-one (VI) [% (m/m)] | n.d.[1] | n.d. | n.d. | n.d. | n.d. |
| (S)-AHB acid methylester amide (IX) [% m/m)] | n.d. | n.d. | n.d. | n.d. | n.d. |
| Sum of all impurities [% m/m)] | 1.3 | 1.7 | 1.0 | 1.0 | 1.1 |

(continued)

| | | | | | |
|---|---|---|---|---|---|
| Method (V) B | | | | | |
| Chloride [% (m/m)] | 20.8 | 20.9 | 21.1 | 20.8 | 20.9 |

Table 5:

| Batch | 6 | 7 | 8 | 9 |
|---|---|---|---|---|
| Identity (HPLC) | complies | complies | complies | complies |
| Content [% (m/m)] | 97.5 | 98.1 | 97.8 | 97.7 |
| Method (V) A | | | | |
| (2S)-4-amino-2-hydroxy-butanoic acid (IV) [% (m/m)] | 1.0 | 1.1 | 1.0 | 1.4 |
| (S)-AHB acid isopropylester (VIII) [% (m/m)] | 0.11 | 0.09 | 0.11 | 0.10 |
| | | | | |
| Method (V) B | | | | |
| (3S)-3-hydroxypyrrolidin-2-one (VI) [% (m/m)] | n.d. | n.d. | n.d. | n.d. |
| (S)-AHB acid methylester amide (IX) [% (m/m)] | n.d. | n.d. | n.d. | n.d. |
| Sum of all impurities [% (m/m)] | 1.2 | 1.2 | 1.1 | 1.5 |
| Chloride [% (m/m)] | 20.8 | 20.9 | 20.9 | 20.9 |
| n.d.: not detected | | | | |

**Example 3b: (3S)-3-hydroxypyrrolidin-2-one (VI)**

Table 6 shows the yield of the first commercial production campaign of (3S)-3-hydroxypyrrolidin-2-one (VI), started from 100 kg methyl (2S)-4-amino-2-hydroxy-butanoate hydrochloride (V) and 111.2 kg trisodium phosphate:

Table 6:

| Batch | Yield [kg] | Yield [% th.] |
|---|---|---|
| 1 | 44.5 | 74.7 |
| 2 | 36.7 | 61.6 |
| 3 | 45.1 | 75.7 |
| 4 | 43.8 | 73.5 |
| 5 | 49.1 | 82,4 |
| 6 | 47.1 | 79.0 |

A yield of 61.6 to 82.4 % th. was obtained, this corresponds to an average yield of 74.5 % th.. Tables 7 shows the analytical results of these batches.

Table 7:

| Batch | 1 | 2 | 3 | 4 | 5 | 6 |
|---|---|---|---|---|---|---|
| Identity (HPLC) | complies | complies | complies | complies | complies | complies |
| Content [% (m/m)] | 98.4 | 98.4 | 98.0 | 98.3 | 98.4 | 98.3 |
| Method (VI) A | | | | | | |
| (S)-AHB acid methylester amide (IX) [% (m/m)] | n.d. | n.d. | n.d. | n.d. | n.d. | n.d. |

(continued)

| Method (VI) B | | | | | | |
|---|---|---|---|---|---|---|
| Methyl (2S)-4-amino-2-hydroxy-butanoate hydrochloride (V) [% (m/m)]) | n.d. | n.d. | n.d. | n.d. | n.d. | n.d. |
| (S)-4-Amino-2-hydroxybutanoic acid Dimer (X) [% (m/m)] | 0.18 | 0.11 | 0.12 | 0.10 | 0.16 | 0.13 |
| (2S)-4-amino-2-hydroxy-butanoic acid (IV) [% (m/m)] | 0.07 | <0.05 (0.0418) | 0.05 | <0.05 (0.0391) | 0.06 | 0.06 |
| (S)-AHB acid isopropylester (VIII) [% (m/m)]) | n.d. | n.d. | n.d. | n.d. | n.d. | n.d. |
| Sum of all impurities [% (m/m)]) | 0.3 | 0.2 | 0.2 | 0.2 | 0.3 | 0.2 |

**Example 3c: Pure (3S)-pyrrolidin-3-ol hydrochloride (II)**

Table 8 shows the yield of the first commercial production campaign of (3S)-pyrrolidin-3-ol hydrochloride (II), started from 66 kg (3S)-3-hydroxypyrrolidin-2-one (VI) and 776 kg Lithium aluminum-hydride, 2.4 M in THF.

Table 8:

| Batch | Yield [kg] | Yield [% th.] |
|---|---|---|
| 1 | 59.5 | 73.8 |
| 2 | 64.2 | 79.6 |
| 3 | 59.6 | 73.9 |
| 4 | 63.5 | 78.7 |
| 5 | 57.9 | 71.8 |
| 6 | 59.7 | 74.0 |

A yield of 71.8 to 79.6 % th. was obtained, this corresponds to an average yield of 75.3 % th..

Table 9 shows the analytical results of these batches:

Table 9:

| Batch | 1 | 2 | 3 | 4 | 5 | 6 |
|---|---|---|---|---|---|---|
| Identity (GC) | complies | complies | complies | complies | complies | complies |
| Chemical purity [% (area-%)] Method (II) A | > 99.5 | > 99.5 | > 99.5 | > 99.5 | > 99.5 | > 99.5 |
| Optical purity [%ee] Method (II) B | > 99.9 | > 99.9 | > 99.9 | > 99.9 | > 99.9 | > 99.9 |

**Example 3d: Pure (3S)-pyrrolidin-3-ol (I)**

Table 10 shows the yield of the first commercial production campaign (3S)-pyrrolidin-3-ol (I), started from 20.0 kg (S)-Pyrrolidin-3-ol hydrochloride (II), 23.3 kg aqueous 25% sodium hydroxide and 26.5 kg trisodium phosphate.

Table 10:

| Batch | Yield [kg] 1st distillation | Yield [% th.] 1st distillation | Yield [kg] sump distillation | Yield [% th.] sump distillation | Overall yield [kg] | Overall yield [% th.] |
|---|---|---|---|---|---|---|
| 1 | 10.6 | 75.2 | 1.1 | 7.8 | 11.7 | 83.0 |

(continued)

| Batch | Yield [kg] 1st distillation | Yield [% th.] 1st distillation | Yield [kg] sump distillation | Yield [% th.] sump distillation | Overall yield [kg] | Overall yield [% th.] |
|---|---|---|---|---|---|---|
| 2 | 8.9 | 63.1 | 2.4 | 17.0 | 11.3 | 80.1 |
| 3 | 9.7 | 68.8 | 2.8 | 19.9 | 12.5 | 88.7 |
| 4 | 10.0 | 70.9 | 1.9 | 13.5 | 11.9 | 84.4 |
| 5 | 11.1 | 78.7 | 1.3 | 9.2 | 12.4 | 87.9 |
| 6 | 10.4 | 73.8 | 1.7 | 12.1 | 12.1 | 85.8 |
| 7 | 9.2 | 65.3 | 2.2 | 15.6 | 11.4 | 80.9 |

A yield of 80.1 to 88.7 % th. was obtained, this corresponds to an average yield of 84.4 % th..

Tables 11 and 12 show the analytical results of these batches. Batch numbers are provided for the first distillation and the corresponding sump distillation in the two tables.

Table 11:

| Batch (1st distillation) | 1 | 2 | 3 | 4 | 5 | 6 | 7 |
|---|---|---|---|---|---|---|---|
| Identity (GC) | complies | complies | complies | complies | complies | complies | complies |
| Chemical purity [% (area-%)] Method (I) A | 100.00 | 99.99 | 99.99 | 100.00 | 99.98 | 99.94 | 99.94 |
| Optical purity [%ee] Method (I) B | 99.99 | 100.00 | 99.99 | 99.98 | 100.00 | 100.00 | 100.00 |
| (S)-Pyrrolidin-3-ol open ring dimer (XI) [% (area-%)] Method (I) A | n.d. | n.d. | n.d. | n.d. | n.d. | n.d. | n.d. |
| 4-Amino-1,2-butanediol (XII) [% (area-%)] Method (I) A | n.d. | n.d. | n.d. | n.d. | n.d. | n.d. | n.d. |
| Any unspecified impurity [% (area-%)] Method (I) A | n.d. | 0.01 | 0.01 | n.d. | 0.01 | 0.01 | 0.04 |
| Water content [% (m/m)] Method (I) C | 0.23 | 0.13 | 0.07 | 0.05 | 0.02 | 0.06 | 0.09 |
| Content (i.d.s.) [% (m/m)] Method (I) C | 99.9 | 99.7 | 99.7 | 99.7 | 99.9 | 99.8 | 99.9 |

Table 12:

| Batch (sump distillation) | 1 | 2 | 3 | 4 | 5 | 6 | 7 |
|---|---|---|---|---|---|---|---|
| Identity (GC) | complies | complies | complies | complies | complies | complies | complies |
| Chemical purity [% (area-%)] Method (I) A | 99.99 | 100.00 | 99.98 | 99.99 | 99.97 | 99.98 | 99.98 |
| Optical purity [%ee] Method (I) B | 100.00 | 99.99 | 99.98 | 99.98 | 100.00 | 100.00 | 100.00 |

(continued)

| Batch (sump distillation) | 1 | 2 | 3 | 4 | 5 | 6 | 7 |
|---|---|---|---|---|---|---|---|
| (S)-Pyrrolidin-3-ol open ring dimer (XI) [% (area-%)] Method (I) A | n.d. | n.d. | n.d. | n.d. | n.d. | n.d. | n.d. |
| 4-Amino-1,2-butanediol (XII) [% (area-%)] Method (I) A | n.d. | n.d. | n.d. | n.d. | n.d. | n.d. | n.d. |
| Any unspecified impurity [% (area-%)] Method (I) A | 0.01 | n.d. | 0.01 | n.d. | 0.01 | 0.01 | 0.02 |
| Water content [% (m/m)] Method (I) C | 0.12 | 0.31 | 0.11 | 0.09 | 0.13 | 0.10 | 0.16 |
| Content (i.d.s.) [% (m/m)] Method (I) C | 99.8 | 99.8 | 99.7 | 99.9 | 99.9 | 99.9 | 100.0 |

**Example 4**

Production of Larotrectinib sulfate

7 batches of Larotrectinib sulfate shown in Table 13 were manufactured using pure (3S)-pyrrolidin-3-ol (I) obtained according to the present invention. In no batch the Aminodiol impurity of formula (XIII) or the Open-ring Dimer impurity of formula (XIV) could be detected.

Table 13:

| Batch | 1 | 2 | 3 | 4 | 5 | 6 | 7 |
|---|---|---|---|---|---|---|---|
| Aminodiol impurity (XIII) [% (area-%)] | n.d. | n.d. | n.d. | n.d. | n.d. | n.d. | n.d. |
| Open-ring Dimer impurity (XIV) [% (area-%)] | n.d. | n.d. | n.d. | n.d. | n.d. | n.d. | n.d. |
| n.d.: not detected | | | | | | | |

The following HPLC method (= method (III) A) was used to determine the impurities Aminodiol of formula (XIII) and the Open-ring Dimer of formula (XIV) in samples of Larotrectinib sulfate:
Stationary phase: Waters Xselect CSH Phenyl-Hexyl (100 mm, 4.6 mm ID, 3.5 μm particle size); mobile phase A: 1.0 mL trifluoroacetic acid / 1 L water; mobile phase B: acetonitrile; diluent: water: acetonitrile 50:50 (v/v); sample solution: approximately 25 mg sample accurately weighed are dissolved in 100 mL of diluent; UV detection at 264 nm; oven temperature: 40 °C; injection volume: 10 μL; flow: 1.5 mL/min; linear gradient after 1.5 minutes isocratic run (5 % B) in 2 steps: 5 % B -> 95 % B (21 min), 1.5 minutes holding time at 95 % B, 95 % B -> 5 % B (0.1 min); the column is equilibrated at 5 % B for 3 minutes at 5 % B prior to each run; relevant potential by-products:

Open-ring Dimer impurity (XIV) at RRT 0.83 (RT=7.1 min);
Aminodiol impurity (XIII) at RRT 0.95 (RT=8.2 min)

The following relative response factors (RRF) are used:

- Open-ring Dimer impurity (XIV): 0.97
- Aminodiol impurity (XIII): 0.86

**Comparative example**

As comparative example the synthesis is provided of (S)-3-hydroxypyrrolidine (the free base) according to US

7, 652, 152 B2 (examples 8 and 9).

**Preparation of (S)-3-hydroxy-2-pyrrolidinone**

47.64 g 4-amino-(S)-2-hydroxybutylic acid (0.4 mol) was dissolved in 192.20 g methanol (6.0mol) contained in a 500 mL round-bottom flask, 39.22 g sulfuric acid (1 equivalent) was added thereto at room temperature. When 4-amino-(S)-2-hydroxybutylic acid was completely dissolved, the reaction mixture was heated to 80° C (bath temperature) and agitated under reflux for 4 hours to complete the reaction. Thereafter, 4-amino-(S)-2-hydroxybutylic acid methylester hydrogensulfate was confirmed by NMR (a sample was evaporated to an oily residue)

$^1$H-NMR (600 MHz, $D_2O$): δ 4.83 (s, 9-10 H), 4.47 (dd, 1H), 3.78 (s, 3H), 3.15 (t, 2H), 2.17-2.34 (m, 1H), 1.94-2.14 (m, 1H).

After the reaction mixture was cooled down to room temperature, 23.8 g water and 30.4 g potassium carbonate (1.1 equivalent) were added thereto and agitated at room temperature for 16 hours to obtain (S)-3-hydroxy-2-pyrrolidinone. Inorganic substances, which were precipitated by adding 96.12 g methanol to a residue obtained after filtering and concentrating the reaction mixture, were removed by filtering the reaction mixture twice, and a filtrate thus obtained was concentrated under reduced pressure to obtain (S)-3-hydroxy-2-pyrrolidinone (yield: 86.3%).

$^1$H-NMR (600 MHz, DMSO-$d_6$): δ 7.65 (bs, 1H), 5.3-5.6 (bs, 1H), 3.98 (t, 1H), 3.1-3.2 (m, 2H), 2.2-2.3 (m, 1H), 1.65-1.8 (m, 1H).

**Preparation of (S)-3-hydroxypyrrolidine**

(S)-3-Hydroxy-2-pyrrolidinone - (0.1 mol, 10.11 g), diglyme (1.13 mol, 151.65 g) and $NaBH_4$(0.4 mol, 15.13 g) were added to a 500ml round-bottom flask at room temperature and sulfuric acid (20.2 g) was gently dropped thereto for 1 hour. After the addition, the mixture was heated to 80° C (inner temperature) and kept for 42 hours to complete the reaction. When the reaction was completed, 79.1 g methanol was added thereto to inactivate the reaction, and the reaction mixture was neutralized with 10.60 g 25% aqueous hydrochloric acid (72.68 mmol). Then, pH of the reaction mixture was adjusted to 11 or more by using an 50% aqueous solution of sodium hydroxide (20 g, 250 mmol), and salts precipitated therefrom were removed through filtration.

A resulting filtrate was concentrated under reduced pressure. The residue was tried to distill under reduced pressure (150°C, 2-4 mbar). Unfortunately, no product was distilled over. The residue remains in the flask. Mass spectroscopy showed that there were only traces of the target compound in the residue.

## Claims

1. Process for preparing pure (3S)-Pyrrolidin-3-ol of formula (I) or pure (3S)-Pyrrolidin-3-ol hydrochloride of formula (II) comprising the steps

   a) acid-catalyzed esterification of aminohydroxybutyric acid of formula (IV)

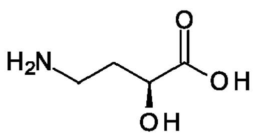

(IV)

   with methanol to the methylester hydrochloride of formula (V)

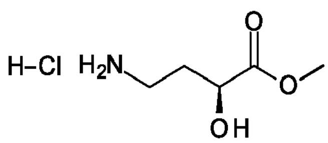

(V)

   b) base-induced ring closure reaction of the methyl ester hydrochloride (V) to the lactam of formula (VI)

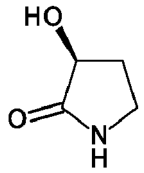

(VI)

c) reducing the amide function in the lactam of formula (VI) to obtain pure (3S)-Pyrrolidin-3-ol hydrochloride of formula (II)

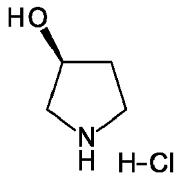

(II)

d) desalination of pure (3S)-Pyrrolidin-3-ol hydrochloride of formula (II) to obtain pure (3S)-Pyrrolidin-3-ol of formula (I)

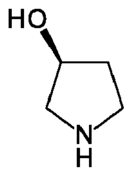

(I)

**characterized in that** in

in step a) hydrochloride acid is used as acid and the methylester hydrochloride of formula (V) is crystallized from isopropanol and isolated
in step b) sodium phosphate is used as base and the lactam of formula (VI) is crystallized from isopropanol and isolated
in step c) the reduction is performed with lithium aluminum hydride in tetrahydrofuran, followed by purification steps of the crude material when the reaction is complete comprising the substeps

c1) addition of sodium hydroxide solution and water
c2) removal of aluminum salts by filtration and washing the aluminum salts with a mixture of tetrahydrofuran and isopropanol
c3) salt formation and crystallization with hydrochloric acid in isopropanol

in step d) the desalination is performed with an inorganic base whereas the ratio ((3S)-Pyrrolidin-3-ol hydrochloride) to (inorganic base) is more than 1.1 eq and less than 2.5 eq. followed by purification steps of the crude product comprising the substeps:

d1) removal of water by azeotropic distillation using ethanol or isopropanol
d2) filtration to remove inorganic salt
d3) repeating substeps d1) and d2) until the water content is < 0.5 % (m/m) determined by Karl-Fischer titration
d4) addition of methyl tert-butyl ether, 2-methyl-, THF, methylcyclopentyl ether and/or diisopropyl ether as anti-solvent for precipitation of inorganic salts
d5) cooling to -10 to 20 °C and filtration to remove inorganic salts
d6) distillation until (3S)-Pyrrolidin-3-ol is obtained as oil
d7) vacuum distillation of the oil using distillation parameters which allow an overdistillation of (3S)-Pyrrolidin-3-ol.

**2.** Pure (3S)-Pyrrolidin-3-ol of formula (I)

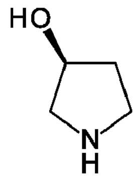

(I)

having

- a chemical purity determined by GC according to method (I) A of not less than 99.80% (area-%)
- an optical purity determined by GC according to method (I) B of not less than 99.9% (enantiomeric excess %ee)
- a content on anhydrous basis determined by potentiometric titration and Karl-Fischer titration according to method (I) C of not less than 99.0%

and containing

- unspecified impurities determined by GC according to method (I) A in an amount less than 0.10% (area-%)
- (S)-Pyrrolidin-3-ol open ring dimer of formula (IX)

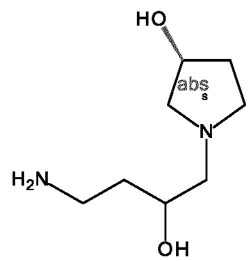

(XI)

determined by GC according to method (I) A in an amount less than 0.05 % (area-%)
- 4-Amino-1,2-butanediol of formula (XII)

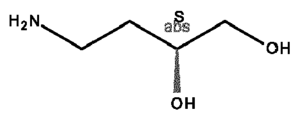

(XII)

determined by GC according to method (I) A in an amount less than 0.05 % (area-%)

**3.** (3S)-Pyrrolidin-3-ol of formula (I) according to claim 2 which is substantially free of (S)-Pyrrolidin-3-ol open ring dimer of formula (XI).

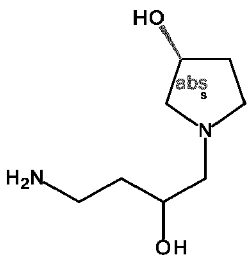

(XI)

determined by GC according to method (I) A.

**4.** (3S)-Pyrrolidin-3-ol of formula (I) according to claim 2 or 3 which is substantially free of 4-Amino-1,2-butanediol of formula (XII)

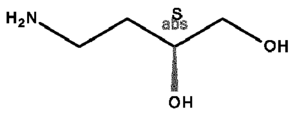

(XII)

determined by GC according to method (I) A.

**5.** Pure (3S)-Pyrrolidin-3-ol hydrochloride of formula (II)

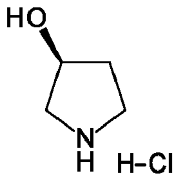

(II)

having

- a chemical purity determined by GC according to method (II) A of not less than 99.5% (area-%)
- an optical purity (enantiomeric excess %ee.) determined by GC according to method (II) B of not less than 99.9% (enantiomeric excess)

**6.** Process for preparing pure Larotrectinib of formula (IIIa)

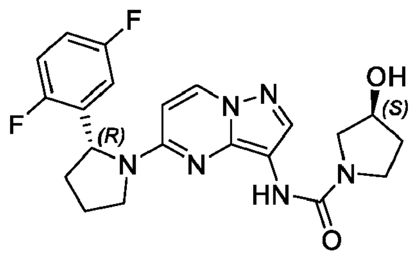

(IIIa)

or a salt thereof,
containing the impurities of formula (XIII) and (XIV)

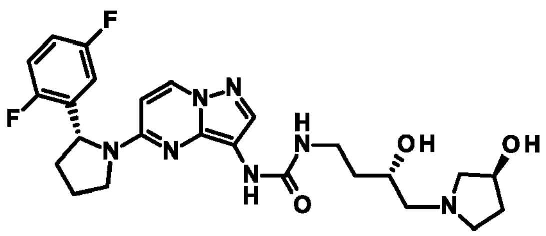

(XIII)

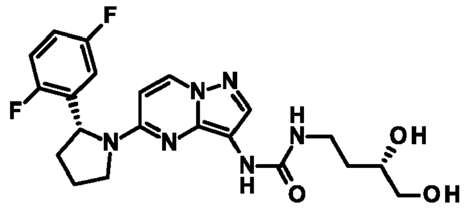

(XIV)

determined by HPLC according to method (III) A each in an amount less than 0.05 % (area-%) by treating a compound of formula 13

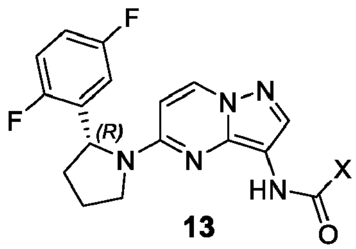

13

or a salt thereof, wherein X is phenoxy with (3S)-Pyrrolidin-3-ol of formula (I)

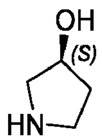

(I)

or a salt thereof to form Larotrectinib or a salt thereof whereas the compound of formula (I) is obtained by a process according to claim 1.

**7.** Process according to claim 6 for preparing a pure sulfate salt of Larotrectinib of formula (IIIb)

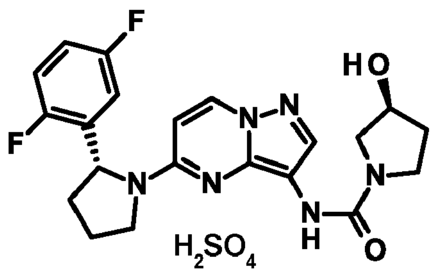

(IIIb

**8.** Process according to claim 6 for preparing pure Larotrectinib of formula (IIIa) or a salt thereof which is substantially free of each of the impurities of formula (XIII) and (XIV) determined by HPLC according to method (III) A.

**9.** Process according to claim 7 for preparing a pure sulfate salt of Larotrectinib of formula (IIIb) which is substantially free of each of the impurities of formula (XIII) and (XIV) determined by HPLC according to method (III) A.

**10.** Pure Larotrectinib of formula (IIIa)

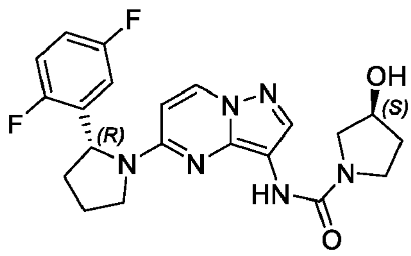

(IIIa

or a salt thereof,
containing the impurities of formula (XIII) and (XIV)

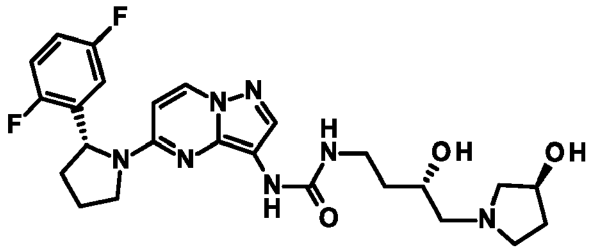

(XIII)

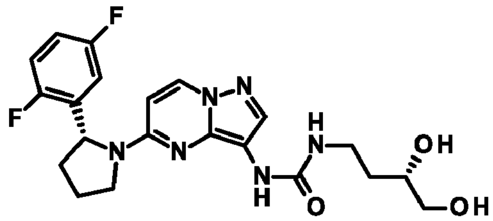

(XIV)

determined by HPLC according to method (III) A each in an amount less than 0.05 % (area-%).

**11.** Pure Salt of Larotrectinib according to claim 9, whereas the salt is a sulfate salt of Larotrectinib of formula (IIIb)

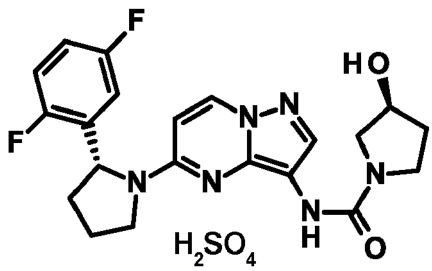

(IIIb

**12.** Pure Larotrectinib of formula (IIIa) or a salt thereof according to claim 9 or a pure sulfate salt of Larotrectinib of formula (III b) according to claim 10 which is substantially free of each of the impurities of formula (XIII) and (XIV) determined by HPLC according to method (III) A.

**13.** The use of pure (3S)-Pyrrolidin-3-ol of formula (I) according to anyone of the claims 2 to 4 or prepared by the process according to claim 1 for preparing Larotrectinib according to formula (IIIa) or a salt thereof

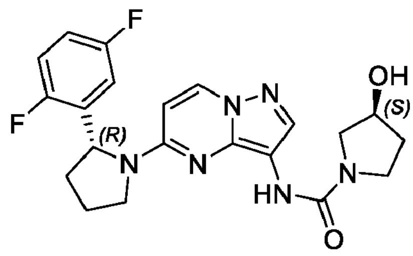

(IIIa

for a step in which the compound of formula 13

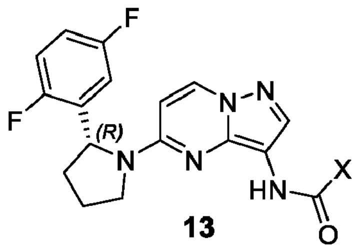

13

or a salt thereof, wherein X is phenoxy
is treated with pure (3S)-Pyrrolidin-3-ol of formula (I)

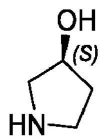

(I)

or a salt thereof to form Larotrectinib or a salt thereof.

**14.** Use according to claim 12 for preparing a sulfate salt of Larotrectinib of formula (IIIb)

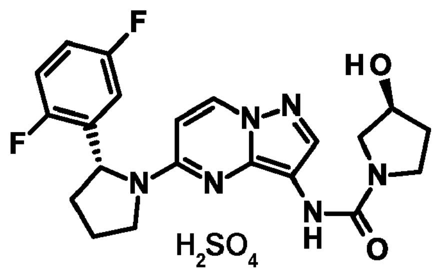

(IIIb

**15.** Pharmaceutical composition comprising pure Larotrectinib of formula (IIIa) or a salt thereof according to claim 9 or 11 or a pure sulfate salt of Larotrectinib of formula (IIIb) according to claim 10 or 11.

Europäisches Patentamt
European Patent Office
Office européen des brevets

# EUROPEAN SEARCH REPORT

Application Number
EP 22 21 1952

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2007/011162 A1 (CHIROAD INCORPORATE [KR]; DONGWOO FINE CHEM CO LTD [KR] ET AL.) 25 January 2007 (2007-01-25) * paragraph [0075] - paragraph [0082] * * examples 8, 9 * ----- | 1,6-9, 12,13 | INV. C07D487/04 C07D207/12 |
| A,D | WO 2017/201241 A1 (REYNOLDS MARK [US]; EARY CHARLES TODD [US] ET AL.) 23 November 2017 (2017-11-23) * claim 1 * ----- | 1,6-9, 12,13 | |
| | | | TECHNICAL FIELDS SEARCHED (IPC) |
| | | | C07D |

~~The present search report has been drawn up for all claims~~

1

| Place of search | Date of completion of the search | Examiner |
|---|---|---|
| Munich | 10 May 2023 | Miniejew, Catherine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 22 21 1952

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**1, 6-9(completely); 12, 13(partially)**

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 22 21 1952

The Search Division considers that the present European patent application does not comply with the requirements of unity of invention and relates to several inventions or groups of inventions, namely:

**1. claims: 1, 6-9(completely); 12, 13(partially)**

**subject-matter for preparing pure (3S)-Pyrrolidin-3-ol of formula (I) or its hydrochloride salt of formula (II)**

---

**2. claims: 2-4(completely); 12, 13(partially)**

**subject-matter related to (3S)-Pyrrolidin-3-ol of formula (I) characterized by a certain purity degree measured by GC and Karl-Fischer titration**

---

**3. claim: 5**

**subject-matter related to (3S)-Pyrrolidin-3-ol hydrochloride of formula (II) characterized by a certain purity degree measured by GC**

---

**4. claims: 10, 11, 14**

**subject-matter related to Larotrectinib of formula (IIIa) or its salt thereof containing the impurities of formulas (XIII) and (XIV) in a certain amount**

---

## ANNEX TO THE EUROPEAN SEARCH REPORT ON EUROPEAN PATENT APPLICATION NO.

EP 22 21 1952

This annex lists the patent family members relating to the patent documents cited in the above-mentioned European search report. The members are as contained in the European Patent Office EDP file on
The European Patent Office is in no way liable for these particulars which are merely given for the purpose of information.

10-05-2023

| Patent document cited in search report | Publication date | Patent family member(s) | Publication date |
|---|---|---|---|
| WO 2007011162 A1 | 25-01-2007 | KR 20070010794 A | 24-01-2007 |
| | | US 2008214837 A1 | 04-09-2008 |
| | | WO 2007011162 A1 | 25-01-2007 |
| WO 2017201241 A1 | 23-11-2017 | AU 2017268371 A1 | 06-12-2018 |
| | | BR 112018073504 A2 | 26-03-2019 |
| | | CA 3024603 A1 | 23-11-2017 |
| | | CL 2018003264 A1 | 21-06-2019 |
| | | CN 110049987 A | 23-07-2019 |
| | | CO 2018013015 A2 | 20-08-2019 |
| | | DK 3458456 T3 | 14-12-2020 |
| | | DK 3800189 T3 | 31-07-2023 |
| | | EP 3458456 A1 | 27-03-2019 |
| | | EP 3800189 A1 | 07-04-2021 |
| | | ES 2836222 T3 | 24-06-2021 |
| | | FI 3800189 T3 | 31-07-2023 |
| | | HR P20201984 T1 | 16-04-2021 |
| | | HU E053643 T2 | 28-07-2021 |
| | | IL 262964 A | 31-12-2018 |
| | | JP 2019516795 A | 20-06-2019 |
| | | KR 20190039474 A | 12-04-2019 |
| | | LT 3458456 T | 25-02-2021 |
| | | PE 20190437 A1 | 27-03-2019 |
| | | PL 3458456 T3 | 19-04-2021 |
| | | PT 3458456 T | 07-12-2020 |
| | | PT 3800189 T | 27-07-2023 |
| | | RU 2018144557 A | 18-06-2020 |
| | | SG 11201810245W A | 28-12-2018 |
| | | SI 3458456 T1 | 30-04-2021 |
| | | US 2019218222 A1 | 18-07-2019 |
| | | WO 2017201241 A1 | 23-11-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010048314 A1
- WO 2017201241 A1
- EP 160451 A
- JP SHO61267577 A
- JP SHO6163652 A
- WO 8808845 A
- US 4916141 A
- EP 391169 A
- EP 304087 A
- EP 398720 A
- EP 366327 A
- WO OI19817 A
- JP 2001220372 B
- WO 9743256 A
- JP 05255204 B
- WO 2003097594 A
- WO 2000015610 A
- JP 60104061 B
- JP 57056457 B
- EP 431521 A
- EP 347818 A
- US 7652152 B2
- WO 2017192605 A1, Zeng, Dexing
- WO 2017192598 A1, Zeng, Dexing
- WO 2015026792 A1, Wang, Guangyi
- US 20110092698 A1, Aebi, Johannes
- WO 2001062714 A1, Cheshire, David
- CN 1869008 A, Zhang, Wen
- KR 915666 B1, Noh, Gyeong Rok
- WO 2007011162 A1, Roh, Kyoung Rok
- EP 430234 A2, Satomi
- CN 104098502 A, Xiao, Xun
- WO 2015043364 A1, Gao, Renlong
- WO 2016164828 A1, Almutairi, Adah
- WO 2014008285 A1, Bjornson, Kyla
- WO 2011013141 A2, Kharul, Rajendra
- US 20150284362 A1, Bersot, Ross


### Non-patent literature cited in the description


- *J. Med. Chem.,* 1986, vol. 29, 2504-2511
- *Heterocycles,* 1986, vol. 24 (5
- *Tetrahedron Lett.,* 1984, vol. 25, 2793
- *J. Org. Chem.,* 1992, vol. 57, 4352-4361
- *J. Med. Chem.,* 1994, vol. 37, 2138-2144
- *Synlett,* 1995, 55-57
- *Syn. Comm.,* 1994, vol. 24, 1381-1387
- *J. of Med. Chem.,* 1993, vol. 3, 72-80
- *Syn. Comm.,* 1993, vol. 23, 2691-2699
- *J. Chem. Soc. Perkin Trans.,* 1993, vol. 1, 1421-1424
- *Bioorganic & Medicinal Chemistry Letters,* vol. 2, 827
- *Syn. Commun. 15,* (985), 587-598
- *Synthesis,* 2010, (20), 3423-3428
- *Journal of Organic Chemistry,* 1967, vol. 32 (8), 2388-91
- **GAI, YONGKANG et al.** *Bioconjugate Chemistry,* 2016, vol. 27 (3), 515-520
- **SRAIRI, DRISS et al.** *Bulletin de la Societe Chimique de France,* 1987, (2), 297-301
- **WONG, CHI HUEY et al.** *Journal of Organic Chemistry,* 1985, vol. 50 (11), 1992-4
- **RONG, HAO-JIE et al.** *Journal of Organic Chemistry,* 2017, vol. 82 (1), 532-540
- **DAVIES, CHRISTOPHER D. et al.** *Synlett,* 2008, (13), 2028-2032
- **JIANG, CHAOZHE et al.** *Cuihua Xuebao,* 2005, vol. 26 (4), 263-264
- **OLEJNICZAK, JASON et al.** *Macromolecules (Washington, DC, United States),* 2015, vol. 48 (10), 3166-3172
- **HUANG, PEI-QIANG et al.** *Heterocycles,* 2003, vol. 60 (8), 1833-1841
- **BENTLEY, JONATHAN M. et al.** *Journal of the Chemical Society, Chemical Communications,* 1995, (2), 231-2
- **PIRES, RAUL et al.** *Tetrahedron,* 1997, vol. 53 (27), 9213-9218
- **KAMAL, AHMED et al.** *Tetrahedron: Asymmetry,* 2003, vol. 14 (17), 2587-2594